# EUROPEAN PATENT SPECIFICATION

(11) **EP 1 212 058 B1**
(45) Date of publication and mention of the grant of the patent: **23.08.2006**
(21) Application number: 00961801.8
(22) Date of filing: 12.09.2000
(51) Int. Cl.: A61K 31/422, A61K 31/424, A61K 9/00, A61K 47/32

(54) **TOPICAL TREATMENT FOR PREVENTION OF OCULAR INFECTIONS**
TOPISCHE BEHANDLUNG ZUR VORBEUGUNG VON AUGENINFEKTIONEN
TRAITEMENT TOPIQUE UTILISE DANS LA PREVENTION D'INFECTIONS OCULAIRES

(30) Priority: 13.09.1999 US 394617; 11.09.2000 US 659063
(43) Date of publication of application: 12.06.2002
(73) Proprietor: INSITE VISION INCORPORATED, Alameda, CA 94501 (US)
(72) Inventor: BOWMAN, Lyle, M., Pleasanton, CA 94566 (US); SAMIR, Roy, San Ramon, CA 94583 (US); SHEN, Peng, Hayward, CA 94542 (US)
(74) Representative: Wolf, Matthias
(86) International application number: PCT/US2000/024914
(87) International publication number: WO 2001/019366

(56) References cited:
- EP-A- 0 352 781
- WO-A-00/03710
- WO-A-00/18387
- WO-A-99/25344

## Description

### 2. Field of the Invention

The present invention relates to a method for treating or preventing infections in the eye and to compositions useful therein.

### 3. Description of the Related Arts

The eye is susceptible to bacterial and parasitic infections arising from both traumatic and non-traumatic related events. Infections are a concern after ocular surgery and precautions are correspondingly taken to prevent the onset of infection. However, even without the invasive trauma of a surgical procedure, infections in the eye lids, conjunctiva, cornea, and other ocular tissues can arise.

Treating infections in ocular tissues can be challenging and/or problematic because of the difficulty in delivering an antibiotic to the affected tissue. In general, ocular ' infections are treated by local injection, systemic administration, or topical application of an antibiotic. The route of administration depends on the antibiotic selected, the location of the infection and the type of infection.

The simple and direct approach of topically applying the antibiotic to the exterior of the eye has several benefits, including the avoidance of side effects and the reduced chance of developing resistant strains of bacteria as compared to systemic administration. However, for a variety of reasons, many antibiotics are not amenable or suitable for topical application to the eye.

For example, in order for a topical application to be effective, the antibiotic must be able to penetrate the desired tissue. This may include penetrating the conjunctiva and the cornea. Also, the penetration rate must be sufficient to impart an effective dose. Many drugs do not possess a requisite penetration ability with regard to the tissues of the eye. It should be noted that the external, layers of the eye are quite different from the tissues encountered in the stomach and intestinal tract. Thus, while a certain drug may be readily absorbed in the intestines and introduced into the blood supply for systemic administration, the same drug may be incapable of being absorbed by, or of passing through, the substantially avascular outer layers of the conjunctiva or cornea at a minimally acceptable therapeutic concentration. The mechanism of transport or uptake of the drug is entirely different for topical administration than for oral administration.

Another concern is that the antibiotic will be toxic to the eye. A toxic response includes redness, swelling and/or discharge. Toxicity is especially problematic for topical administration because it is a concentration dependent phenomenon. The concentration ratio between tear fluid and ocular tissue in topical administration is generally in the range of about 1:500 to 1:1000, due to the penetration gradient. Thus, while a drug may be nontoxic at the minimum effective concentration, the 500% to 1000% increase in concentration associated with topical administration may well induce a toxic response. Again, the fact that oral or systemic administration shows the drug to be compatible with ocular tissue does not predict or address the toxicity issue associated with topical administration.

A further potential unsuitability of an antibiotic is the practicality of topical administration by the patient. Assuming that sufficiently high concentrations of the antibiotic can be used to achieve an effective dose within the target tissue without a toxic response, the application may nonetheless be irritating. An irritation response includes temporary burning, stinging and/or watering of the eye. Beyond whether the increased watering of the eyes washes away so much of the antibiotic composition that an effective dose is prevented, the patient may simply be resistant to complying with the dosage regimen because of the irritation. By failing to comply with the dosing regimen, the treatment efficacy is reduced or eliminated.

Some antibiotics have been found to sufficiently meet the above requirements so as to be applicable to topical administration. Examples of antibiotics that are reported to be useful in ocular topical administration include tobramycin, gentamycin, fluoroquinolone derivatives including norfloxacin, ofloxacin, and ciprofloxacin, naphthyridine, tetracyclines, and erythromycin. However, in view of the rise in resistant strains of bacteria, it would be desirable to find additional antibiotics that can be topically applied to the eye. It would be further desirable to provide an ophthalmic topical formulation that is effective against gram-positive aerobic bacteria as well as anaerobic organisms.

### SUMMARY OF THE INVENTION

The present invention relates to an aqueous polymeric suspension comprising water, 0,05% to 5,0% of an oxazolidinone antibiotic, 0,1 to 10% of a polymeric suspending agent and a solubility enhancer, wherein said polymeric suspending agent comprises a water-swellable water-insoluble crosslinked carboxy-vinyl polymer.

The invention also relates to an aqueous polymeric suspension comprising water, 0,05% to 5.0% of an oxazolidinone antibiotic, 0.1 to 10% of a polymeric suspending agent and a solubility enhancer, wherein said polymeric suspending agent comprises a polycarbophil.

Further the present invention relates to a composition comprising water, a polymeric suspending agent, a solubility enhancer and a compound of formula III: 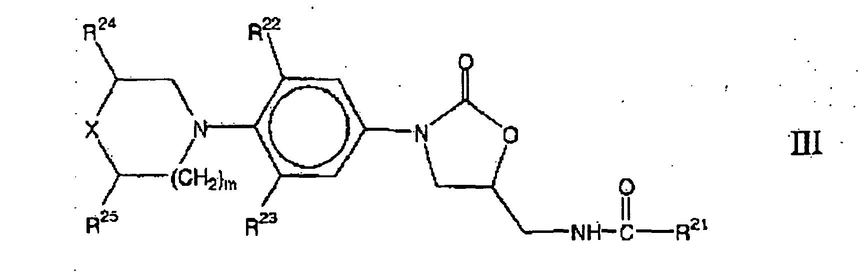 wherein
- X: is O, S, SO, SO₂SNR⁴⁰ or S(O)NR⁴⁰
- R²¹: is hydrogen, C₁-C₆ alkyl unsubstituted or substituted with one or more of the following: F, Cl, hydroxy, C₁-C₈ alkoxy, C₁-C₈ acyloxy or -O-CH₂-phenyl; C₁-C₆ cycloalkyl, amino; C₁-C₈ alkyamino; C₁-C₈ dialkylamino or C₁-C₈ alkoxy;
- R²⁴: is H; CH₃ CN, CO₂H, CO₂R, or (CH₂)ₘR⁴¹ except when X is not O then R²⁴ is H;
- R²²: and R²³ are each and independently H, F, or Cl;
- R²⁵: is H except when X is O and R²⁴ is CH₃ then R²⁵ can be H or CH₃;
- R⁴⁰: is independently H, C₁-C₄ alkyl (unsubstituted or substituted with chloro; fluoro, hydroxy, C₁-C₈ alkoxy, amino, C₁-C₈ alkylamino, or C₁-C₈ dialkyamino) or p-toluenesulfonyl;
- R⁴¹: is hydrogen, OH, OR²¹, OCOR²¹, NH₂, NHCOR²¹ or N(R⁴⁰)₂ and m is 0, 1 or 2

### DETAILED DESCRIPTION OF THE INVENTION

"Oxazolidinone antibiotic" as used herein means a compound having an oxazolidinone nucleus that exhibits antimicrobial activity. Typically the compound nucleus is substituted as shown in the following structure fragment: 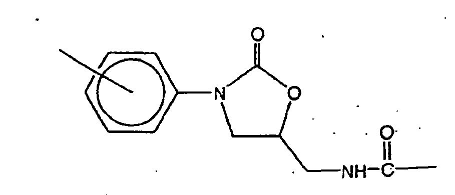 The phenyl ring can be substituted by a variety of groups as is well known in the oxazolidinone antibiotic art and can form a fused ring with the substituents and/or the oxazolidinone ring itself. The carbonyl moiety is bonded to one of a number of well known terminal groups. An "phenyloxazolidinone" means oxazolidinone ring with a phenyl group bonded to the ring nitrogen a shown in the above fragment, with or without the amidemethylene substituent.

More specifically, the oxazolidinone antibiotics used in the present invention can generally be represented by the following formulas I and II and the pharmaceutically acceptable salts thereof: 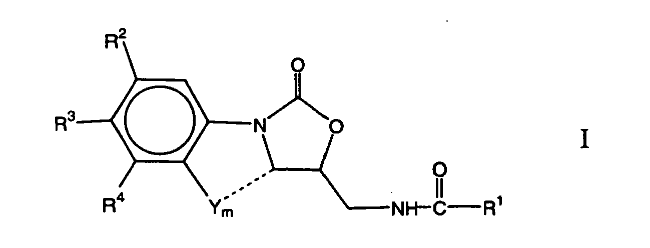 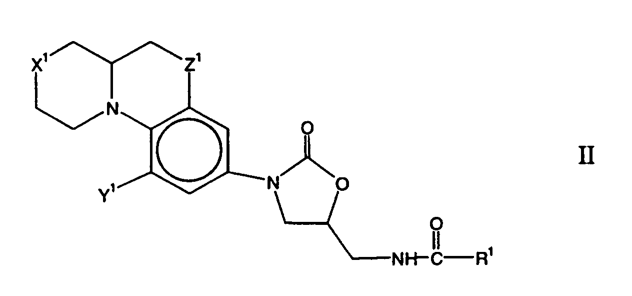 wherein:
- R¹: is selected from the group consisting of (1) -H, (2) NH₃, (3) C₁-C₈ alkylamine, (4) C₁₋C₈ dialkylamine, (5) C₁-C₈ alkoxy, (6) C₁-C₁₂ alkyl optionally substituted with 1-3 Cl, (7) C₃-C₁₂ cycloalkyl, (8) C₅-C₁₂ alkenyl containing one double bond, (9) C₁-C₈ acyloxy, (10) O-CH²-phenyl, (11) phenyl optionally substituted with 1-3 -OH, -OCH₃, -OC₂H₅, -NO₂, -F, -Cl, -Br, -COOH and SO_{3H}, -N(R¹⁻¹)(R¹⁻²);
where R¹⁻¹ and R¹⁻² are the same or different and are -H, C₁-C₅ alkyl, (12) furanyl, (13) tetrahydrofuranyl, (14) 2-thiophene, (15) pyrrolidinyl, (16) pyridinyl, (17) -OR¹⁻³,
where R¹⁻³ is C₁-C₄ alkyl,
(18) -NH₂, (19) -NHR¹⁻⁴,
where R¹⁻⁴ is C₁-C₃ alkyl or phenyl,
(20) -NR¹⁻⁴R¹⁻⁵,
where R¹⁻⁵ is C₁-C₃ alkyl and R¹⁻⁴ is as defined above, and where R¹⁻⁴ and R¹⁻⁵ can be taken together with the attached nitrogen atom to form a saturated mono-nitrogen C₅-C₇ heterocyclic ring including O,
(21) CH₂-OH, and (22) CH₂-OR¹⁻⁶,
where R¹⁻⁶ is C₁-C₄ alkyl, or CO-R¹⁻⁷ is C₁-C₄ alkyl or -phenyl;
- R² and R⁴: are the same or different and are selected from the group consisting of (1) H, (2) -OH, (3) halogen, (4) CF₃, (5) OCH₃ provided that only one of R² or R⁴ is -H, and (6) O-CO-R²⁻¹,
where R²⁻¹ is C₁-C₃ alkyl or -phenyl;
- R³: is selected from the group consisting of (1) H, (2) halogen, (3) -O-CH₃, (4) -O-C₂H₅, (5) piperdine, (6) 4-hydroxypyridine, (7) piperazine, unsubstituted or substituted with Q
where Q is a 5, 6-, 7- or 8-membered heterocyclic ring of the general formula -C*H=CH-CH=Z-Y²=W* where the atoms or symbols representing an atom marked with an asterisk (*) are bonded to each other resulting in the formation of a ring where one of Z, Y² and W is -N= and the others are C=, unsubstituted or substituted with substituents selected from the group consisting of H, -C₁-C₄ alkyl, -NO₂, -NH₂, -NH-CO-[C₁-C₄ alkyl], -CN, -COOH, -O-[C₁-C₄ alkyl], halogen and N-oxides thereof; or Q is C₁-C₄ alkyl unsubstituted or substituted with substituents selected from the group consisting of hydrogen, methyl, ethyl, isopropyl, tert-butyl, benzyl, phenyl, pyridyl, acetyl, difluoroacetyl, hydroxyacetyl, benzoyl, methoxy carbonyl, ethoxy carbonyl, 2-chloroethoxy carbonyl, 2-hydroxyethoxy carbonyl, 2-benzyloxyethyoxy carbonyl, 2-methoxyethoxy carbonyl, 2,2,2-trifluroethoxy carbonyl, cyanomethyl, 2-cyanoethyl, carbomethoxymethyl, 2-carbomethoxyethyl, 2-fluoroethoxy carbonyl, benzyloxy carbonyl, tertiary-butoxy carbonyl, methyl sulfonyl, phenyl sulfonyl, or paratoluenesulfonyl,
(8) phenyl, (9) pyridyl, (10) pyrazidyl, (11) pyridazinyl, (12) pyrimidinyl, (13) 1,2,3-triazinyl, (14) 1,2,4-triazinyl, (15) 1,2,5-triazinyl, (16) quinolinyl, (17) isoquinolinyl, (18) quinoxalinyl, (19) quinazolinyl, (20) phthalazinyl, (21) cinnolinyl, (22) naphthylpyridinyl, (23) indolyl having nitrogen optionally substituted with R³¹⁻¹, (24) pyrrolopyridinyl having the saturated nitrogen substituted with R³¹⁻¹ (25) furanopyridinyl, (26) thienopyridinyl, (27) benzothiazolyl, (28) benzoxazolyl, (29) imidazolyl having the saturated nitrogen substituted with R³¹⁻¹, (30) pyrazolyl having the saturated nitrogen substituted with R³¹⁻¹, (31) thiazolyl, (32) isothiazolyl, (33) oxazolyl, (34) isoxazolyl, (35) pyrrolyl having nitrogen substituted with R³¹⁻¹ (36) furanyl, (37) thiophenyl wherein substituents 1-37 are optionally substituted with U and V, (38) 1,2,3-triazolyl, (39) 1,2,4-triazolyl having the saturated nitrogen substituted with R³¹⁻¹.
where R³¹⁻¹ is H, C₁-C₄ alkyl unsubstituted or substituted with one or more halogens, C₃-C₆ cycloalkyl, or C(O)R³¹⁻²,
where R³¹⁻² is -H, C₁-C₄ alkyl unsubstituted or substituted with one or more halogens, or phenyl unsubstituted or substituted with one or more halogens;
wherein 1,2,3-triazolyl and 1,2,4-triazolyl are unsubstituted or substituted with U;
each occurrence of V is independently selected from substituents selected from the group consisting of H, halogen, R³⁻¹, OR³⁻¹;
where R³⁻¹ is -H or C₁-C₄ alkyl or NO₂;
each occurrence of U is independently selected from the group consisting of (1) H, (2) C₁-C₈ alkyl unsubstituted or substituted with one or more substituents selected from the group consisting of halogens, -OH, =O other than at alpha position, S(O)ₙR³⁻²,
where R³⁻² is C₁-C₄ alkyl or C₃-C₈ cycloalkyl and NR³⁻³R³⁻⁴,
where R³⁻³ and R³⁻⁴ are the same or different and are selected from the group consisting of H, C₁-C₈ alkyl, C₃-C₈ cycloalkyl, -(CH₂)ᵣCHOR³⁻⁵, and (CH₂)ᵣNR³⁻⁶R³⁻⁷, or where R³⁻³ and R³⁻⁴ taken together are selected from the group consisting of (CH₂)O(CH₂), (CH₂)ₜCH(CO)R³⁻⁸, and (CH₂)N(R³⁻⁸)(CH₂)₂,
where R³⁻⁵ is H, or C₁-C₄ alkyl, or R³⁻⁶ and R³⁻⁷ are the same or different
and are H, or C₁-C₄ alkyl or taken together are (CH₂)ᵣ;
(3) C₂-C₅ alkenyl, (4) C₃-C₈ cycloalkyl, (5) OR³⁻³ where R³⁻³ is as defined above, (6) -CN, (7) S-(O)ₙ-R³⁻⁸
where R³⁻⁸ is C₁-C₄ alkyl unsubstituted or substituted with one or more substituents selected from the group consisting of halogens, OH, CN, NR³⁻³R³⁻⁴ where R³⁻³ and R³⁻⁴ are as defined above, and CO₂R³⁻⁵ where R³⁻⁵ is as defined above, C₂-C₄ alkenyl, NR³⁻⁹R³⁻¹⁰,
where R³⁻⁹ is H, C₁-C₄ alkyl, or C₃-C₈ cycloalkyl and R³⁻¹⁰ is -H, C₁-C₄ alkyl, C₁-C₄ alkenyl, C₃-C₄ cycloalkyl, -OR³⁻⁵, or NR³⁻⁶R³⁻⁷, where R³⁻⁵, R³⁻⁶ and R³⁻⁷ are as defined above; N₃, NHC(O)R³⁻¹¹;
where R³⁻¹¹ is C₁-C₄ alkyl optionally substituted with one or more halogens,
(8) -S(O)₂-N=S(O)ᵢR³⁻¹⁴R³⁻¹⁵,
where R³⁻¹⁴ and R³⁻¹⁵ are the same or different and are C₁-C₂ alkyl or taken together are (CH₂)_{q}-,
(9)-S-C(O)-R³⁻¹¹ where R³⁻¹¹ is as defined above; (10) tetrazoyl, (11) NR³⁻³R³⁻⁴ where R³⁻³ and R³⁻⁴ are as defined above, (12) N(R³⁻³)COR³⁻¹¹ where R³⁻³ and R³⁻¹¹ are as defined above; (13) -N(R³⁻³)-S-(O)ₙR³⁻¹¹ where R³⁻³ and R³⁻¹¹ are as defined above, (14) -CONR³⁻³R³⁻⁴
where R³⁻³ and R³⁻⁴ are as defined above,
(15) -C(O)R³⁻¹⁶
where R³⁻¹⁶ is -H, C₁-C₈ alkyl optionally substituted with -OR³⁻⁵, -OC(O)R³⁻⁵, NR³⁻³R³⁻⁴, S(O)ₙR³⁻¹⁷, C₃-C₈ cycloalkyl, or C₂-C₅ alkenyl optionally substituted with -CHO or -CO₂R³⁻⁵, where R³⁻³, R³⁻⁴ and R³⁻⁵ are as defined above and
where R³⁻¹⁷ is C₁-C₄ alkyl or C₃-C₈ cycloalkyl,
(16) -C(=NR³⁻¹⁸)R³⁻¹⁶ where R³⁻¹⁶ is as defined above and
where R³⁻¹⁸ is -NR³⁻³R³⁻⁴, -OR³⁻³ or NHC(O)R³⁻³ where R³⁻³ and R³⁻⁴ are as defined above,
(17) CR³⁻¹⁶(OR³⁻¹⁹)OR³⁻²⁰
where R³⁻¹⁶ is as defined above and R³⁻¹⁹ and R³⁻²⁰ are the same or different and are C₁-C₄ alkyl or taken together are -(CH₂)ⱼ-,
(18) -C(NR³⁻³R³⁻²¹)(R³⁻¹⁶)(R³⁻⁹), (19) -C(OR³⁻⁵)(R³⁻¹⁶)(R³⁻⁴), (20)-C(O-C(O)R³⁻⁵)(R³⁻¹⁶)(R³⁴), (21) -C(OR³⁻⁵)((CH₂)ₜ₋NR³⁻³(R³⁻⁴)(R³⁻⁴)), (22) -C(NR³⁻³R³⁻²¹)(R³⁻¹⁶)(R³⁻⁹),
where R³⁻³, R³⁻⁴, R³⁻⁹, and R³⁻¹⁶ are as defined above and where R³⁻²¹ is R³⁻⁴ or -NR³⁻⁴R³⁻⁵ where R³⁻⁴ and R³⁻⁵ are as defined above;
with the proviso that at least one of the R², R³ and R⁴ is H,
- X¹: is selected from the group consisting of NR¹⁵, CR¹⁶R¹⁷, S, SO, SO₂ or O; R¹⁵ is selected from the group consisting of (1) -H, (2) C₃-C₆ alkyl, (3) -(CH₂)p-Aryl, (4) -(CH₂)ₚ-C(=O)-R¹⁵⁻¹, (5)-C(=O)-R¹⁵⁻¹,(6) -C(=O)-(CH₂)ₚ-C(=O)R¹⁵⁻¹, (7) SO₂₋R¹⁵⁻³, (8) (C=O)-Het, (9) 2-pyridyl, (10) 2-pyrimidinyl, (11) 3-pyridazinyl or (12) 2-quinolyl;
where R¹⁵⁻¹ is selected from the group consisting of -H, C₁-C₆ alkyl, Aryl, (CH₂)ₚ₋Aryl, or -(CH₂)ₚ-OR¹⁵⁻²;
where R¹⁵⁻² is selected from the group consisting of -H, C₁-C₆ alkyl, Aryl, (CH₂)ₚ-Aryl, or -C(=O)-C₁-C₆ alkyl;
where R¹⁵⁻³ is C₁-C₆ alkyl, or Aryl;
Aryl is phenyl, unsubstituted or substituted with one or more substituents selected from the group consisting of halogen, C₁-C₆ alkyl, C₁-C₄ alkoxy, C₁-C₄ alkylthio, -OH, -NH₂, SH, -NO₂ or -O-C(=O)-OCH₃;
Het is a 5-, 6-, 8-, 9-, or 10-membered heteroaromatic moiety having one or more atoms selected from the group consisting of N, O, and S;
R¹⁶ and R¹⁷ are each and independently selected from the group consisting of (1) -H, (2) C₁-C₆ alkyl, (3) C₁-C₆ alkoxy, (4) C₁-C₆ alkylthio, (5) -(CH₂)ₙ-OR¹⁶⁻¹, (6) NR¹⁶⁻²R¹⁷⁻², (7) -N=CH-NR¹⁶⁻³R^{17-3,} (8) -C(=O)-NR¹⁶⁻²R¹⁷⁻², (9) (CH₂)ₙ-C(=O)-R¹⁶⁻⁴, and (10) (CH₂)ₙ-Q¹, -(CH₂)ₙ-W¹;
where R¹⁶⁻¹ is -H, C₁-C₆ alkyl, -(CH₂)ₙ-OR¹⁶⁻⁵, -(CH₂)ₙ-C(=O)-R¹⁶⁻⁴, -C(=O)-(CH₂)ₙ-OR¹⁶⁻³, or tosyl;
where R¹⁶⁻² and R¹⁷⁻² are each and independently selected from the group consisting of -H, C₁-C₆ alkyl, -(CH₂)ₙ-OR¹⁶⁻⁵, -C(=O)-R¹⁶⁻⁴, -C(=O)-NR¹⁶⁻⁵R¹⁷⁻⁵, -(CH₂)ₚ-Aryl, and -Het;
where R¹⁶⁻⁵ and R¹⁷⁻⁵ are each and independently selected from the group consisting of -H, C₁-C₆ alkyl or methoxymethyl;
where R¹⁶⁻³ and R¹⁷⁻³ are each and independently selected from the group consisting of -H, C₁-C₆ alkyl, -C(=O)-R¹⁶⁻⁴ and (CH₂)ₚ-Aryl;
where R¹⁶⁻⁴ is selected from the group consisting of -H, -OH, C₁-C₆ alkyl, C₁-C₆ alkoxy, -O-CH₂-O-C(=O)-R¹⁶⁻⁵ and -(CH₂)ₙ-C(=O)-OR¹⁶⁻⁵;
Q' is a saturated 5-membered heterocyclic moiety having 1-2 atoms selected from the group consisting of N, O, and S;
W¹ is a saturated 6-membered heterocyclic moiety having 1-2 atoms selected from the group consisting of N, O, and S;
or where R¹⁶ and R¹⁷ taken together are =O, =NR¹⁷⁻⁴, =S, =CR¹⁶⁻³R¹⁷⁻³ or Q¹;
where R¹⁷⁻⁴ is selected from the group consisting of -OR¹⁶⁻¹, C₁-C₆ alkyl, C₁-C₆ alkoxy, and -(CH₂)p-Aryl;
- Y¹: is H or halogen;
- Yₘ: Y: is CR⁷ and m is 0,1, or 2,
when m is 0, the Y moiety is not present, and therefore there is no central ring in formula I (i.e., the dotted line in formula I represents no bond);
when m is 1, Y forms a tricyclic-fused 5-membered ring with carbon 3 of the oxazolidinone ring, and R⁷ is either one or two moieties,
when R⁷ is two moieties comprising R⁷⁻¹ and R⁷⁻², each is bound to the same carbon in the tricyclic-fused 5-membered ring, which in turn is bound to carbon 3 of the oxazolidinone ring (i.e., the dotted line in formula I is a single bond), andR⁷⁻¹ and R⁷⁻² are each independently H, C₁-C₃ alkyl, Cl, Br, OH, or I;
when R⁷ is one moiety, either:
there is a double bond between the carbon atom to which R⁷ is attached and carbon 3 of the oxazolidinone ring (i.e., the dotted line in formula I is a double bond), or
there is a single bond between the carbon atom to which R⁷ is attached and carbon 3 of the oxazolidinone ring (i.e., the dotted line in formula I is a single bond), and R⁷ is -C(=O)H;
and when m is 2, Yₘ is CR⁷CR⁸, which forms a tricyclic-fused 6-membered ring with carbon 3 of the oxazolidinone ring, and R⁷ and R⁸ are each either one or two moieties,
when R⁷ and R⁸ are both one moiety, a double bond is formed between the carbon atoms that comprise Y to which they are attached, and R⁷ and R⁸ are both H,
when R⁷ and R⁸ are both two moieties, R⁷ comprises R⁷⁻¹ and R⁷⁻², where one of R⁷⁻¹ and R⁷⁻² is H and the other is H, OH, or OC(=O)R⁷⁻³, where R⁷⁻³ is C₁-C₃ alkyl or phenyl, optionally substituted with 1 or 2 F, Cl, OH, or OCH₃, and R⁸ comprises R⁸⁻¹ and R⁸⁻², where one of R⁸⁻¹ and R⁸⁻² is H and the other is H, OH, or OC(=O)R⁸⁻³, where R⁸⁻³ is C₁-C₃ alkyl or phenyl, optionally substituted with 1 or 2 F, Cl, OH, or OCH₃;
- Z¹: is -0, -N, -S, -SO₂, SO, NR¹⁸, -SNR⁹⁻¹, or S(O)NR⁹⁻¹,
where R¹⁸ is selected from the group consisting of -H, C₁-C₆ alkyl, -C(=O)-R¹⁸⁻¹, -C(=O)-OR¹⁸⁻¹ and C(=O)-(CH₂)ₚ-C(=O)R¹⁸⁻¹;
where R¹⁸⁻¹ is H, C₁-C₆ alkyl, -(CH₂)ₚ-Aryl, or -(CH₂)ₚ-OR¹⁸⁻²;
where R¹⁸⁻² is -H, C₁-C₆ alkyl, or (CH₂)p-Aryl;
where R⁹⁻¹ is independently -H, C₁-C₄ alkyl (optionally substituted with -Cl, -F, -OH, C₁-C₄ alkoxy, NH₃, C₁-C₈ alkylamine or C₁-C₈ dialklylamine), -p-toluenesulfonyl, C(R⁹R¹⁰)
where R⁹ and R¹⁰ are each and independently -H, -C₁-C₈ alkyl, -C₁-C₈ alkoxy, -C₁-C₈ alkylthio, -(CH₂)ₘ-OR⁵¹, -O-(CH₂)ₘ-OR⁵¹, NR⁴²R⁵², -N=CH-NR⁴⁴R⁵⁵, -C(=O)-NR⁴⁴R⁵⁵, -C(=O)-NR⁴²R⁵² or -(CH₂)ₘ-C(=A¹)-R⁴¹, or they may combine together to form =O, =NR⁴³, =S, CR⁴⁴R⁵⁴, or an optionally substituted, unsaturated or saturated 5- or 6-membered hetero ring having 1-3 hetero atoms selected from the group consisting of a nitrogen atom, an oxygen atom and a sulfur atom;
R⁴¹ is -H, -(CH²)ₘ-OH, C₁-C₈ alkyl, C₁-C₈ alkoxy, -O-CH₂-O-C(=O)-R¹¹, or -(CH₂)ₘ-C(=O)-OR¹¹;
where R¹¹ is -H, -H, C₁-C₈ alkyl, methoxymethyl,
R⁴² and R⁵² are each and independently -H, -(CH₂)ₘ-OR¹¹, C₁-C₈ alkyl, -C(=O)-R⁴¹, C(=O)-NR¹¹R¹², -(CH₂)ₚ-phenyl, thiazol-2-yl, or they may combine together to form a pyrrolidino group, a piperidino group, a piperazino group, a morpholino group, or a thiomorphinolino group, each of may be substituted by C₁-C₄ alkyl or -(CH₂)ₘ OH;
where R¹¹ and R¹² are each independently -H, C₁-C₈ alkyl, methoxymethyl,
R₄₃ is -H, -OR⁵¹, C₁-C₈ alkyl, C₁-C₈ alkoxy, -(CH₂)ₚ-phenyl, -NR⁴²R⁵², -NH-C(=NH)-NH₂, [1,2,4]triazol-4-yl, or cyano;
R⁴⁴ and R⁵⁴ are each and independently -H, C₁-C₈-alkyl, -C(=O)-R⁴¹, or -(CH₂)ₚ₋phenyl;
R⁵¹ is -H, C₁-C₈ alkyl, C₁-C₈ alkyl substituted by one or more hydroxy, C₂-C₈ alkenyl, C₁-C₈ halogenoalkyl, -(CH₂)ₘ-OR¹¹, -(CH₂)ₘ C(=O)-R₄₁, -C(=O)-(CH₂)ₘ-OR⁴⁴ or tosyl;
R⁹ optionally is COOR⁹⁻² where R⁹⁻² is -C(O)-R⁹⁻³, -PO₃ or -P(O)(OH)₂; where R⁹⁻³ is C₁-C₆ alkyl, -N(R⁹⁻⁴)₂, C₁-C₆ alkyl-N(R⁹⁻⁴)₂, -phenyl-N(R⁹⁻⁴)₂, -phenyl-NHC(O)CH₂NH₂, -C₂H₄-, morpholinyl, pyridinyl, C₁-C₆ alkyl-OH, C₁-C₆ alkyl-OCH₃, C₁-C₆ allcyl-C(O)CH₃, -O-C₁-C₆ alkyl-OCH₃, C₁-C₃ alkyl-piperazinyl (optionally substituted with C₁-C₃ alkyl), imidazolyl, C₁-C₆ allcyl-COOH, -C(CH₂OH)₂CH₃, where R⁹⁴ is -H or C₁-C₆ alkyl; -(CH₂)ₚ -R⁹⁻⁵ where R⁹⁻⁵ is -H, -CH₃, CH₂CH₃, isopropyl, tert-butyl, phenyl, pyridyl, acetyl, difluoroacetyl, hydroxyacetyl, benzoyl, methoxy carbonyl, ethoxy carbonyl, 2,2,2-trifluoroethoxy carbonyl, cyanomethyl, 2-cyanoethyl, carbomethoxymethyl, 2-carbomethoxyethyl, 2-fluoroethoxy carbonyl, benzyloxy carbonyl, tertary-butoxy carbonyl, methyl sulfonyl, phenyl sulfonyl, or paratoluenesulfonyl;
R⁹ optionally is a five membered heterocyclic ring (azoyl) of the general form C*H=N-D-B-A*- where the atoms or symbols representing an atom marked with an asterisk (*) are bonded to each other resulting in the formation of a ring,
wherein A, B, and D are independently oxygen, nitrogen, sulfur or carbon, in all cases the piperazine nitrogen is attached to the carbon atom of the carbon-nitrogen double bond;
- A¹: is oxygen atom or ethyleneketal;
- i's: are each and independently 0 or 1;
- j's: are each and independently 2 or 3;
- k's: are each and independently 1 or 2;
- m's: are each and independently 0, l,or 2;
- n's: are each and independently 0,1, or 2;
- p's: are each and independently 1, 2, 3 or 4;
- q's: are each and independently 3, 4, or 5;
t's are each and independently 1, 2, or 3.

In one aspect of the present invention, the oxazolidinone antibiotic is a compound of the formula III: 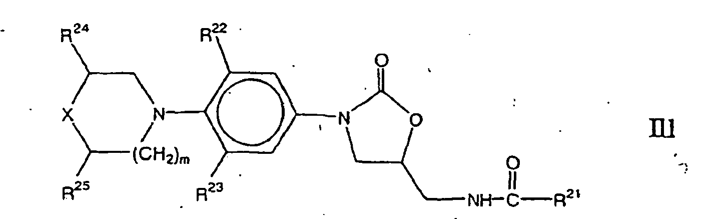
- X: is O, S, SO, SO₂SNR⁴⁰ or S(O)NR⁴⁰
- R²¹: is hydrogen, C₁-C₆ alkyl unsubstituted or substituted with one or more of the following F, Cl, hydroxy, C₁-C₈ alkoxy, C₁-C₈ acyloxy or-O-CH₂-phenyl; C₁-C₆ cycloalkyl, amino, C₁-C₈ alkyamino, C₁-C₈ dialkylarnina or C₁-C₈ alkoxy;
- R²⁴: is H; CH₃, CN, CO₂H CO₂R, or (CH₂)ₘR⁴¹ except when X is not O then R²⁴ is H;
- R²²: and R²³ are each and independently H, F, or Cl;
- R²⁵: is H except when X is O and R²⁴ is CH₃ then R²⁵ can be H or CH₃;
- R⁴⁰: is independently H, C₁-C₄ alkyl (unsubstituted or substituted with chloro, fluoro, hydroxy, C₁-C₈ alkoxy, amino, C₁-C₈ alkylamino, or C₁-C₈ dialkyamino) or p-toluenesulfonyl;
- R⁴¹: is hydrogen, OH, OR²¹, OCOR²¹, NH₂, NHCOR²¹ or N(R⁴⁰)₂ and m is 0, 1 or 2

The compounds of formula III are described in U.S Patents 5,688,792 and 5,880,118. These compounds are preferred, in part, because of their efficacy against multiply-resistant staphylococci and their low minimum inhibitory concentration values.

Oxazolidinone antibiotics and their synthesis are well known in the art. Examples of oxazolidinone antibiotics and specific synthesis schemes can be found in the following U.S. Patents: 5,929,248, 5,922,707, 5,880,118, 5,837,870, 5,801,246. 5,756,732, 5,736,545, 5,700,799, 5,652,238, 5,688,792, 5,668,286, 5,654,435, 5,654,428, 5,565,571, 5,547,950, 5,247,090, 5,231,188, 4,977,173, 4,948,801, 4,461,773, and 4,340,606, the entire contents of each patent being incorporated herein by reference. A particularly preferred oxazolidinone is a compound of formula IV:

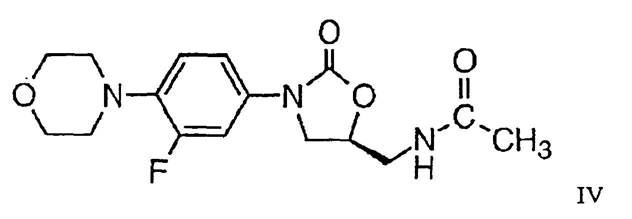

It has now been discovered that oxazolidinone antibiotics in general and the compounds of formulas III. and IV in particular are amenable to topical administration on the eye. The oxazolidinone antibiotic can be supplied to the eye surface as an aqueous ophthalmic suspension. Any technique and ocular dosage form that supplies an oxazolidinone antibiotic to the external eye surface is included within the notion of "topically applying." Although the external surface of the eye is typically the outer layer of the conjunctiva, it is possible that the sclera, cornea or other ocular tissue could be exposed such as by rotation of the eye or by surgical procedure and thus be an external surface.

The amount of oxazolidinone antibiotic topically supplied is effective to treat or prevent infection in a tissue of the eye: This means that the conditions of application result in a retarding or suppression of the infection. Typically at least about MIC₅₀ for the targeted bacteria or parasite is delivered to the ocular tissue by the topical application of an effective amount. More concretely, the concentration within the ocular tissue is desired to be at least about 0.25 µg/g, preferably at least 1 µg/g, and more preferably about 10-50 µg/g. Generally, tissue concentrations that exceed about 50 µg/g, especially greater than 100 µg/g, are nearing or within a toxic response range. The amount of oxazolidinone antibiotic actually supplied to the external eye surface will almost always be much higher than the tissue concentration. This reflects the penetration hold up of the oxazolidinone antibiotic by the outer tissue layers of the eye, the loss of antibiotic due to natural clearing processes and that penetration is to some extent concentration driven. Thus, supplying greater amounts to the exterior will drive more antibiotic into the tissues.

The topical application of an oxazolidinone antibiotic can be used to treat or prevent a variety of conditions associated with ocular infection. For example, conditions of the lids including blepharitis, blepharconjunctivitis, meibomianitis, acute or chronic hordeolum, chalazion, dacryocystitis, dacryoadenities, and acne rosacea; conditions of the conjunctiva including conjunctivitis, ophthalmia neonatorum, and trachoma; conditions of the cornea including corneal ulcers, superficial and interstitial keratitis, keratoconjunctivitis, foreign bodies, and post operative infections; and conditions of the anterior chamber and uvea including endophthalmitis, infectious uveitis, and post operative infections, are a few of the tissues and conditions that can be treated by topical application of an oxazolidinone antibiotic. The prevention of infection includes preoperative treatment prior to surgery as well as other suspected infectious conditions or contact. Examples of prophylaxis situations include treatment prior to surgical procedures such as blepharoplasty, removal of chalazia, tarsorrhapy, procedures for the canualiculi and lacrimal drainage system and other operative procedures involving the lids and lacrimal apparatus; conjunctival surgery including removal of ptyregia, pingueculae and tumors, conjunctival transplantation, traumatic lesions such as cuts, burns and abrasions, and conjunctival flaps; corneal surgery including removal of foreign bodies, keratotomy, and corneal transplants; refractive surgery including photorefractive procedures; glaucoma surgery including filtering blebs; paracentesis of the anterior chamber; iridectomy; cataract surgery; retinal surgery; and procedures involving the extra-ocular muscles. The prevention of ophthalmia neonatorum is also included.

More generally, the oxazolidinone antibiotics can be used to treat or prevent ocular infections caused by a variety of bacteria or parasites, including but not limited to one or more of the following organisms: *Staphylococcus* including *Staphylococcus aureus* and *Staphylococcus epidermidis; Streptococcus* including *Streptococcus pneumoniae* and *Streptococcus pyogenes* as well as *Streptococci* of Groups C, F, and G and Viridans group of *Streptococci; Haemophilus inf luenza* including biotype III *(H. Aegyptius); Haemophilus ducreyi; Moraxella catarrhalis; Neisseria* including *Neisseria gonorrhoeae* and *Neisseria meningitidis; Chlamydia* including *Chlamydia trachomatis, Chlamydia psittaci,* and *Chlamydia pneumoniae; Mycobacterium* including *Mycobacterium tuberculosis* and *Mycobacterium* avium-intracellular complex as well as atypical mycobacterium including *M. marinum, M. fortuitm,* and *M. chelonae; Bordetella pertussis; Campylobacter jejuni; Legionella pneumophila; Bacteroides bivius; Clostridium perfringens; Peptostreptococcus* species; *Borrelia burgdorferi; Mycoplasma pneumoniae; Treponema pallidum; Ureaplasma urealyticum;* toxoplasma; malaria; and nosema.

The oxazolidinone antibiotic is applied to the exterior surface of the eye, usually in an ophthalmically acceptable composition which comprises an ophthalmically acceptable carrier and the oxazolidinone antibiotic. The "ophthalmically acceptable carrier" is used in a broad sense and includes any material or composition that can contain and release the oxazolidinone antibiotic and that is compatible with the eye. Typically the ophthalmically acceptable carrier is water or an aqueous solution or suspension, but also includes oils such as those used to make ointments and polymer matrices such as used in ocular inserts. Generally, oxazolidinone antibiotics are soluble in water. Accordingly, an aqueous solution of an oxazolidinone antibiotic can be formed and used for topical application. A polymeric suspending agent may also be used to provide sustained release of the antibiotic. The polymeric suspending agent may be insoluble, thereby forming a polymeric suspension, or soluble, in which case no polymeric suspension is formed. Oxazolidinone can be present in suspension or solution, or both. One embodiment of the present invention is an aqueous composition comprising a soluble polymeric suspending agent in solution, with oxazolidinone in solution or suspension, or both. Ointments and solid dosage forms can also be used as delivery compositions as are well known in the art.

The concentration of oxazolidinone antibiotic present in the ophthalmic composition depends upon the dosage form, the release rate, the dosing regimen, and the location and type of infection. Generally speaking, the concentration is from about 0.01 to 5%, more typically 0.1 to 2%, for fluid compositions and 0.5 to 50% for solid dosage forms, however, the compositions are not limited thereto.

The fluid ophthalmic compositions of the present invention, including aqueous solutions, ointments and suspensions, have a viscosity that is suited for the selected route of administration. A viscosity in the range of from about 1,000 to 30,000 centipoise is useful for a drop. About 30,000 to about 100,000 centipoise is an advantageous viscosity range for ophthalmic administration in ribbon form. The viscosity can be controlled in many ways known to the worker skilled in the art.

The ophthalmic compositions may contain one or more of the following: surfactants, adjuvants including additional medicaments, buffers, antioxidants, tonicity adjusters, preservatives, thickeners or viscosity modifiers, and the like. Additives in the formulations may desirably include sodium chloride, EDTA (disodium edetate), and/or BAK (benzalkonium chloride), sorbic acid, methyl paraben, propyl paraben, chlorhexidine, glycerin, and sodium perborate. In a preferred embodiment, solutions can include sodium chloride, EDTA (disodium edetate), and/or BAK (benzalkonium chloride), sorbic acid, chlorhexidine, glycerin, and sodium perborate. In another preferred embodiment, ointments can include methyl paraben, propyl paraben, and chlorbutanol.

A further aspect of the present invention involves the above-mentioned use of additional medicaments in combination with the oxazolidinone antibiotic. A composition comprising an oxazolidinone antibiotic, an additional medicament, and an ophthalmically acceptable carrier can advantageously simplify administration and allow for treating or preventing multiple conditions or symptoms simultaneously. The "additional medicaments," which can be present in any of the ophthalmic compositional forms described herein including fluid and solid forms, are pharmaceutically active compounds having efficacy in ocular application and which are compatible with an oxazolidinone antibiotic and with the eye. Typically, the additional medicaments include other antibiotics, antivirals, antifungals, anesthetics, anti-inflammatory agents including steroidal and non-steroidal anti-inflammatories, and anti-allergic agents. Examples of suitable medicaments include aminoglycosides such as amikacin, gentamycin, tobramycin, streptomycin, netilmycin, and kanamycin; macrolides like azithromycin and erythromycin; fluoroquinolones such as ciprofloxacin, norfloxacin, ofloxacin, trovafloxacin, lomefloxacin, levofloxacin, and enoxacin; sulfonamides; polymyxin; chloramphenicol; neomycin; paramomomycin; colistimethate; bacitracin; vancomycin; tetracyclines; rifampin and its derivatives ("rifampins"); cycloserine; beta-lactams; cephalosporins; amphotericins; fluconazole; flucytosine; natamycin; miconazole; ketoconazole; corticosteroids; diclofenac; flurbiprofen; ketorolac; suprofen; comolyn; lodoxamide; levocabastin; naphazoline; antazoline; and pheniramimane. These other medicaments are generally present in a pharmaceutically effective amount as is understood by workers of ordinary skill in the art. These amounts are generally within the range of from about 0.01 to 5%, more typically 0.1 to 2%, for fluid compositions and from 0.5 to 50% for solid dosage forms.

The aqueous ophthalmic compositions (solutions or suspensions) for use in the present invention use water which has no physiologically or ophthalmically harmful constituents. Typically purified or deionized water is used. The pH is adjusted by adding any physiologically and ophthalmically acceptable pH adjusting acids, bases or buffers to within the range of about 5.0 to 8.5. Examples of acids include acetic, boric, citric, lactic, phosphoric, hydrochloric, and the like, and examples of bases include sodium hydroxide, sodium phosphate, sodium borate, sodium citrate, sodium acetate, sodium lactate, tromethamine, THAM (trishydroxymethylamino-methane), and the like. Salts and buffers include citrate/dextrose, sodium bicarbonate, ammonium chloride and mixtures of the aforementioned acids and bases.

The osmotic pressure (π) of the aqueous ophthalmic composition is generally from about 10 milliosmolar (mOsM) to about 400 mOsM, more preferably from 260 to 340 mOsM. If necessary, the osmotic pressure can be adjusted by using appropriate amounts of physiologically and ophthalmically acceptable salts or excipients. Sodium chloride is preferred to approximate physiologic fluid, and amounts of sodium chloride ranging from about 0.01% to about 1% by weight, and preferably from about 0.05% to about 0.45% by weight, based on the total weight of the composition, are typically used. Equivalent amounts of one or more salts made up of cations such as potassium, ammonium and the like and anions such as chloride, citrate, ascorbate, borate, phosphate, bicarbonate, sulfate, thiosulfate, bisulfate, sodium bisulfate, ammonium sulfate, and the like can also be used in addition to or instead of sodium chloride to achieve osmolalities within the above-stated range. Similarly, a sugar such as mannitol, dextrose, sorbitol, glucose and the like can also be used to adjust osmolality.

A preferred form of the present invention provides achieving a sufficiently high tissue concentration with a minimum of doses so that a simple dosing regimen can be used to treat or prevent bacterial or parasitic infections. To this end, a preferred technique involves forming or supplying a depot of oxazolidinone antibiotic in contact with the external surface of the eye. A depot refers to a source of oxazolidinone antibiotic that is not rapidly removed by tears or other eye clearance mechanisms. This allows for continued, sustained high concentrations of oxazolidinone antibiotic to be present in the fluid on the external surface of the eye by a single application. In general, it is believed that absorption and penetration are dependent on both the dissolved drug concentration and the contact duration of the external tissue with the drug-containing fluid. As the drug is removed by clearance of the ocular fluid and/or absorption into the eye tissue, more drug is provided, e.g. dissolved, into the replenished ocular fluid from the depot.

A depot can take a variety of forms so long as the oxazolidinone antibiotic can be provided in sufficient concentration levels therein and is releasable therefrom and that the depot is not readily removed from the eye. A depot generally remains for at least about 30 minutes after administration, preferably at least 2 hours and more preferably at least 4 hours. The term "remains" means that neither the depot composition nor the oxazolidinone antibiotic is exhausted or cleared from the surface of the eye prior to the indicated time. In some embodiments, the depot can remain for up to eight hours or more. Typical ophthalmic depot forms include aqueous polymeric suspensions, ointments, and solid inserts.

Ointments are well known ophthalmic compositions and are essentially an oilbased delivery vehicle. Typical ointments use a petroleum and/or lanolin base to which is added the active ingredient, usually as 0.1 to 2%, and excipients. Common bases include mineral oil, petrolatum and combinations thereof, but oil bases are not limited thereto. An ointment is usually applied as a ribbon onto the lower eye lid. The disadvantage of ointments is that they are difficult to administer, are messy, and uncomfortable/inconvenient to the patient; i.e. temporarily blurred vision is common.

Inserts are another well known ophthalmic dosage form and are comprised of a matrix containing the active ingredient. The matrix is typically a polymer and the active ingredient is generally dispersed therein or bonded to the polymer matrix. The active ingredient is slowly released from the matrix through dissolution or hydrolysis of the covalent bond, etc. In some embodiments, the polymer is bioerodible (soluble) and the dissolution rate thereof can control the release rate of the active ingredient dispersed therein. In another form, the polymer matrix is a biodegradable polymer that breaks down such as by hydrolysis to thereby release the active ingredient bonded thereto or dispersed therein. The matrix and active ingredient can be surrounded with a polymeric coating such as in the sandwich structure of matrix/matrix+active/matrix, to further control release as is well known in the art. The kinds of polymers suitable for use as a matrix are well known in the art. The oxazolidinone antibiotic can be dispersed into the matrix material or dispersed amongst the monomer composition used to make the matrix material prior to polymerization. The amount of oxazolidinone antibiotic is generally from about 0.1 to 50%, more typically about 2 to 20%. The insert can be placed, depending on the location and the mechanism used to hold the insert in position, by either the patient or the doctor and is generally located under the upper eye lid. A variety of shapes and anchoring configurations, if any, are well known in the art. Preferably a biodegradable or bioerodible polymer matrix is used so that the spent insert does not have to be removed. As the biodegradable or bioerodible polymer is degraded, dissolved, or eroded, the trapped oxazolidinone antibiotic is released. Although inserts can provide long term release and hence only a single application of the insert may be necessary, they are generally difficult to insert and are uncomfortable to the patient.

The preferred form is an aqueous polymeric suspension. Here, at least one of the oxazolidinone antibiotic or the polymeric suspending agent is suspended in an aqueous medium having the properties as described above. Typically the oxazolidinone antibiotic is in solution, although it is possible for the oxazolidinone antibiotic to be in suspension or both in solution and in suspension in significant amounts generally no less than 5% in either phase (weak to moderate water solubility and relatively high total concentrations). The polymeric suspending agent is preferably a suspension (i.e. water insoluble and/or water swellable), although water soluble suspending agents are also suitable for use with a suspension of the oxazolidinone antibiotic. The suspending agent serves to provide stability to the composition and to increase the residence time of the dosage form on the eye. It can also enhance the sustained release of the drug in terms of both longer release times and a more uniform release curve.

Examples of polymeric suspending agents include dextrans, polyethylene glycols, polyvinylpyrrolidone, polysaccharide gels, Gelrite®, cellulosic polymers like hydroxypropyl methylcellulose, and carboxy-containing polymers such as polymers or copolymers of acrylic acid, as well as other polymeric demulcents. A preferred polymeric suspending agent is a water swellable, water insoluble polymer, especially a crosslinked carboxy-containing polymer.

Crosslinked carboxy-containing polymers used in practicing this invention are, in general, well known in the art. In a preferred embodiment such polymers may be prepared from at least about 90% and preferably from about 95% to about 99.9% by weight, based on the total weight of monomers present, of one or more carboxy-containing monoethylenically unsaturated monomers (also occasionally referred to herein as carboxy-vinyl polymers). Acrylic acid is the preferred carboxy-containing monoethylenically unsaturated monomer, but other unsaturated, polymerizable carboxy-containing monomers, such as methacrylic acid, ethacrylic acid, β-methylacrylic acid (crotonic acid), cis-α-methylcrotonic acid (angelic acid), trans-α-methylcrotonic acid (tiglic acid), α-butylcrotonic acid, α-phenylacrylic acid, α-benzylacrylic acid, α-cyclohexylacrylic acid, β-phenylacrylic acid (cinnamic acid), coumaric acid (o-hydroxycinnamic acid), umbellic acid (p-hydroxycoumaric acid), and the like can be used in addition to or instead of acrylic acid.

Such polymers may be crosslinked by a polyfunctional crosslinking agent, preferably a difunctional crosslinking agent. The amount of crosslinking should be sufficient to form insoluble polymer particles, but not so great as to unduly interfere with sustained release of the oxazolidinone antibiotic. Typically the polymers are only lightly crosslinked. Preferably the crosslinking agent is contained in an amount of from about 0.01% to about 5%, preferably from about 0.1% to about 5.0%, and more preferably from about 0.2% to about 1%, based on the total weight of monomers present. Included among such crosslinking agents are non-polyalkenyl polyether difunctional crosslinking monomers such as divinyl glycol; 2,3-dihydroxyhexa-1,5-diene; 2,5-dimethyl-1,5-hexadiene; divinylbenzene; N,N-diallylacrylamide; N,N-diallymethacrylamide and the like. Also included are polyalkenyl polyether crosslinking agents containing two or more alkenyl ether groupings per molecule, preferably alkenyl ether groupings containing terminal H₂C=C< groups, prepared by etherifying a polyhydric alcohol containing at least four carbon atoms and at least three hydroxyl groups with an alkenyl halide such as allyl bromide or the like, e.g., polyallyl sucrose, polyallyl pentaerythritol, or the like; see, e.g., Brown U.S. Pat. No. 2,798,053.

Diolefinic non-hydrophilic macromeric crosslinking agents having molecular weights of from about 400 to about 8,000, such as insoluble di- and polyacrylates and methacrylates of diols and polyols, diisocyanate-hydroxyalkyl acrylate or methacrylate reaction products of isocyanate terminated prepolymers derived from polyester diols, polyether diols or polysiloxane diols with hydroxyalkylmethacrylates, and the like, can also be used as the crosslinking agents; see, e.g., Mueller et al. U.S. Pat. Nos. 4,192,827 and 4,136,250.

The crosslinked carboxy-vinyl polymers may be made from a carboxy-vinyl monomer or monomers as the sole monoethylenically unsaturated monomer present, together with a crosslinking agent or agents. Preferably the polymers are ones in which up to about 40%, and preferably from about 0% to about 20% by weight, of the carboxy-containing monoethylenically unsaturated monomer or monomers has been replaced by one or more non-carboxyl-containing monoethylenically unsaturated monomer or monomers containing only physiologically and ophthalmically innocuous substituents, including acrylic and methacrylic acid esters such as methyl methacrylate, ethyl acrylate, butyl acrylate, 2-ethylhexylacrylate, octyl methacrylate, 2-hydroxyethyl-methacrylate, 3-hydroxypropylacrylate, and the like, vinyl acetate, N-vinylpyrrolidone, and the like; see Mueller et al. U.S. Pat No. 4,548,990, the entire contents of which are incorporated herein by reference, for a more extensive listing of such additional monoethylenically unsaturated monomers.

Particularly preferred polymers are lightly crosslinked acrylic acid polymers wherein the crosslinking monomer is 2,3-dihydroxyhexa-1,5-diene or 2,3-dimethylhexa-1,5-diene. Preferred commercially available polymers include polycarbophil (Noveon AA-1) and Carbopol®. Most preferably, a carboxy-containing polymer system known by the tradename DuraSite®, containing polycarbophil, which is a sustained release topical ophthalmic delivery system that releases the drug at a controlled rate, is used in the aqueous polymeric suspension composition of the present invention.

The crosslinked carboxy-vinyl polymers used in practicing this invention are preferably prepared by suspension or emulsion polymerizing the monomers, using conventional free radical polymerization catalysts, to a dry particle size of not more than about 50 µm in equivalent spherical diameter; e.g., to provide dry polymer particles ranging in size from about 1 to about 30 µm, and preferably from about 3 to about 20 µm, in equivalent spherical diameter. Using polymer particles that were obtained by mechanically milling larger polymer particles to this size is preferably avoided. In general, such polymers will have a molecular weight which has been variously reported as being from about 250,000 to about 4,000,000, and from 3,000,000,000 to 4,000,000,000.

In the most preferred embodiment of the invention, the particles of crosslinked carboxy-vinyl polymer are monodisperse, meaning that they have a particle size distribution such that at least 80% of the particles fall within a 10 µm band of major particle size distribution. More preferably, at least 90% and most preferably at least 95%, of the particles fall within a 10 µm band of major particle size distribution. Also, a monodisperse particle size means that there is no more than 20%, preferably no more than 10%, and most preferably no more than 5% particles of a size below 1 µm. The use of a monodispersion of particles will give maximum viscosity and an increased eye residence time of the ophthalmic medicament delivery system for a given particle size. Monodisperse particles having a particle size of 30 µm and below are most preferred. Good particle packing is aided by a narrow particle size distribution.

The aqueous polymeric suspension normally contains 0.05 to 1.0%, preferably 0.1 to 0.5%, more preferably 0.1 to 0.25%, of the oxazolidinone antibiotic and 0.1 to 10%, preferably 0.5 to 6.5% of a polymeric suspending agent. In a further preferred embodiment, which may comprise one or more solubility enhancers, the aqueous polymeric suspension normally contains 0.05 to 5.0%, preferably 0.1 to 2.0%, more preferably 0.2 to 1.50%, of the oxazolidinone antibiotic and 0.1 to 10%, preferably 0.5 to 6.5% of a polymeric suspending agent. In the case of the above described water insoluble, water-swellable crosslinked carboxy-vinyl polymer, a more preferred amount of the polymeric suspending agent is an amount ranging from 0.5 to 2.0%, preferably from 0.5% to about 1.2%, and in certain embodiments from 0.6 to 0.9%, based on the weight of the composition. Although referred to in the singular, it should be understood that one or more species of polymeric suspending agent such as the crosslinked carboxy-containing polymer can be used with the total amount falling within the stated ranges. In one preferred embodiment, the composition contains 0.5 to 1.0%, more preferably 0.6 to 0.8 % of a polycarbophil such as NOVEON AA-1. In another preferred embodiment, the oxazolidinone antibiotic is the compound of formula IV, and the suspension comprises from 0.1 to 5.0% of the compound of formula IV, more preferably 0.2 to 4% of the compound of formula IV, even more preferably 0.3 to 3.5% of the compound of formula IV, and most preferably from 1.0 to 2.0% of the compound of formula IV.

Solubility enhancers may be added to the aqueous compositions above in order to improve the solubility of the oxazolidinone antibiotic. Preferred solubility enhancers include polyethylene glycol, ethoxylated castor oil (trade name, Cremphor), and cyclodextrins such as hydroxypropyl, hydroxyethyl, glucosyl, maltosyl and maltotriosyl derivatives of α-, β-, and γ-cyclodextrin. A particularly preferred solubility enhancer is hydroxypropyl- β cyclodextrin (HPBC), which may be added to further improve the solubility characteristics of any of the aqueous compositions described above. In one embodiment, the composition comprises 0.1% to 20% hydroxypropyl-β-cyclodextrin, more preferably 1% to 15% hydroxypropyl-β-cyclodextrin, and even more preferably from 2.5% to 10% hydroxypropyl-β-cyclodextrin. The amount of solubility enhancer used will depend on the amount of oxazolidinone in the composition, with more solubility enhancer used for greater amounts of oxazolidinone.

In one embodiment, the amount of insoluble lightly crosslinked carboxy-vinyl polymer particles, the pH, and the osmotic pressure can be correlated with each other and with the degree of crosslinking to give a composition having a viscosity in the range of from about 500 to about 100,000 centipoise, and preferably from about 1,000 to about 30,000 or about 1,000 to about 10,000 centipoise, as measured at room temperature (about 25° C) using a Brookfield Digital LVT Viscometer equipped with a number 25 spindle and a 13R small sample adapter at 12 rpm. Alternatively, when the viscosity is within the range of 500 to 3000 centipoise, it may be determined by a Brookfield Model DV-11+, choosing a number cp-52 spindle at 6 rpm.

When water soluble polymers are used as the suspending agent, such as hydroxypropyl methylcellulose, the viscosity will typically be about 10 to about 400 centipoise, more typically about 10 to about 200 centipoises or about 10 to about 25 centipoise.

Aqueous polymeric suspensions of the present invention may be formulated so that they retain the same or substantially the same viscosity in the eye that they had prior to administration to the eye. Alternatively, they may be formulated so that there is increased gelation upon contact with tear fluid. For instance, when a formulation containing DuraSite® or other similar polyacrylic acid-type polymer is administered to the eye at a pH of less than about 6.7, the polymer will swell upon contact with tear fluid since it has a higher pH (around 7). This gelation or increase in gelation leads to entrapment of the oxazolidinone antibiotic, thereby increasing the difficulty for the antibiotic to diffuse out of the composition. All these events eventually lead to increased patient comfort and increased oxazolidinone antibiotic contact time with the eye tissues, thereby increasing the extent of drug absorption and duration of action of the formulation in the eye.

The viscous gels that result from fluid eye drops typically have residence times in the eye ranging from about 2 to about 12 hours, e.g., from about 3 to about 6 hours. The agents contained in these drug delivery systems will be released from the gels at rates that depend on such factors as the drug itself and its physical form, the extent of drug loading and the pH of the system, as well as on any drug delivery adjuvants, such as ion exchange resins compatible with the ocular surface, which may also be present.

The compositions used to topically deliver the oxazolidinone antibiotic of the present invention can be prepared from known or readily available materials through the application of known techniques by workers of ordinary skill in the art without undue experimentation. The oxazolidinone antibiotic can be combined with the other ingredients in the chosen dosage form by conventional methods known in the art.

The oxazolidinone antibiotic-containing composition is topically applied to an eye of a human or non-human animal, the latter including cows, sheep, horses, pigs, goats, rabbits, dogs, cats, and other mammals. The composition can be applied as a liquid drop, ointment, a viscous solution or gel, a ribbon or as a solid. The composition can be topically applied, without limitation, to the front of the eye, under the upper eye lid, on the lower eye lid and in the cul-de-sac. The application can be as a treatment of an infection in the eye or as a preventive such as prior to surgery.

All of the percentages recited herein refer to weight percent, unless otherwise indicated. The following non-limiting examples serve to illustrate certain features of the present invention.

### EXAMPLES 1, 3, 6, 8, and 9

Hydroxypropylmethyl cellulose, sodium chloride, edetate sodium (EDTA), and surfactant are dissolved in a beaker containing approximately 1/3 of the final weight of water and stirred for 10 minutes with an overhead stirred. The drug is added and stirred to disperse for 30 minutes. The solution is sterilized by autoclaving at 121°C. for 20 minutes. Alternately, the drug may be dry heat sterilized and added by aseptic powder addition or sterile filtration if water soluble. Mannitol, Poloxamer 407, BAK, and boric acid are dissolved separately in approximately ½ of the final weight of water and added by sterile filtration (0.22 um filter) and stirred for 10 minutes to form a mixture. The mixture is adjusted to desired pH with 10N sodium hydroxide while stirring, brought to a final weight with water by sterile filtration and aseptically filled into multi-dose containers.

### EXAMPLES 2,4,5,7, and 10

Noveon AA-1 is slowly dispersed into a beaker containing approximately 1/3 of the final weight of water and stirred for 1.5 hrs. with an overhead stirrer. Noveon AA-1 is an acrylic acid polymer available from B.F. Goodrich. Edetate sodium (EDTA), sodium chloride, and surfactant are then added to the polymer solution and stirred for 10 minutes after each addition. The polymer suspension is at a pH of about 3.0-3.5. The drug is added and stirred to disperse for 30 minutes. The mixture is sterilized by autoclaving at 121°C., for 20 minutes. Alternately, the drug may be dry heat sterilized and added by aseptic powder addition or by sterile filtration if the drug is water soluble. BAK, mannitol, and boric acid are dissolved separately in approximately ½ of the final weight of water, added to the polymer mixture by sterile filtration (0.22 um filter) and stirred for 10 minutes. The mixture is adjusted to the desired pH with 10N sodium hydroxide while stirring, brought to final weight with water and by sterile filtration and aseptically filled into multi-dose containers.

| Component | 1 | 2 | 3 | 4 | 5 | 6 | 7 | 8 | 9 | 10 |
|---|---|---|---|---|---|---|---|---|---|---|
| Drug 1 | 0.1 | 0.5 | | | | | | | | |
| Drug 2 | | | 0.1 | .05 | | | | | | |
| Drug 3 | | | | | .5 | | | | | |
| Drug 4 | | | | | | 0.1 | | | | |
| Drug 5 | | | | | | | 0.5 | | | |
| Drug 6 | | | | | | | | 0.1 | | |
| Drug 7 | | | | | | | | | 0.1 | 0.5 |
| NaCl | 0.2 | 0.2 | 0.2 | 0.1 | 0.1 | 0.1 | 0.1 | 0.1 | 0.1 | 0.1 |
| Mannitol | 1.0 | 1.0 | 1.0 | 1.0 | 1.0 | 1.0 | 1.0 | 1.0 | 1.0 | 1.0 |
| Boric Acid | | | | 0.75 | 0.75 | 0.75 | 0.75 | 0.75 | 0.75 | 0.75 |
| EDTA | 0.1 | 0.1 | 0.1 | 0.1 | 0.1 | 0.1 | 0.1 | 0.1 | 0.1 | 0.1 |
| HPMC | 2.0 | | 2.0 | | | 2.0 | | 2.0 | 2.0 | |
| Noveon AA-1 | | 0.8 | | 0.8 | 0.8 | | 0.8 | | | 0.8 |
| BAK | 0.01 | 0.0 1 | 0.01 | 0.01 | 0.01 | 0.01 | 0.01 | 0.01 | 0.01 | 0.01 |
| NaOH | q.s | q.s | q.s | q.s | q.s | q.s | q.s | q.s | q.s | q.s |
| Water | q.s | q.s | q.s | q.s | q.s | q.s | q.s | q.s | q.s | q.s |

| | | | | | | | | | | |
|---|---|---|---|---|---|---|---|---|---|---|
| Drug 1: N-((3-(-fluoro-4-(4-morpholinyl)phenyl)-2-oxo-5-oxazolidinyl)methyl)acetamide (shown in formula IV). | | | | | | | | | | |
| Drug 2: (S)-N-((3-(3-fluoro-4-4-(1-oxothiomorpholin-4-yl)phenyl)-2-oxo-5-oxazolidinyl)methyl)acetamide. | | | | | | | | | | |
| Drug 3: (S)-N-((3-(3-fluoro-4-(3thiazolidinyl)phenyl)-2- oxo-5-oxazolidinyl)methyl)acetamide. | | | | | | | | | | |
| Drug 4: 3-(4-morpholinylpropionic acid, 2-(4-(4-(5-((acetylamino)methyl)-2-oxo-3-oxazolidinyl)-2-fluorophenyl)-1-piperazinyl)-2-oxoethyl ester. | | | | | | | | | | |
| Drug 5: 8-[5-(S)-[(Acetylamino)methyl]-3-oxo-3-oxazolidinyl]-1,2,4a, 5-tetrahydropyrazino[2, 1-c][1,4]benzoxazine-3(4-H)-carboxylic acid phenylmethyl ester. | | | | | | | | | | |
| Drug 6: (S)-1-(4-(5-(acetylamino-methyl)-2-oxo-oxazolidin-3-yl)-2-fluoro-phenyl)-piperidine-4-carboxylic acid ethyl ester. | | | | | | | | | | |
| Drug 7:(S)-N-[(3-[3-Fluoro-4-[4-(2-oxazolyl)-1-piperaziayl]phenyl]-2-oxo-5-oxazolidinyl] methyl] acetamide(1-A). | | | | | | | | | | |

### EXAMPLES 11-32

Examples 11 through 32 are aqueous polymeric suspensions comprising the oxazolidinone compound of formula IV. Examples 11 through 32 are prepared in the following manner: Polycarbophil (Noveon AA-1) is slowly dispersed into a beaker containing approximately 1/3 of the final weight of water and stirred for 1.5 hrs with an overhead stirrer. EDTA, sodium chloride, and surfactant are then added to the polymer solution and stirred for 10 minutes after each addition. The polymer suspension is at a pH of about 3.0-3.5. The compound of formula IV is added and stirred to disperse for 30 minutes. The mixture is sterilized by autoclaving at 121°C., for 20 minutes. Alternately, the drug may be dry heat sterilized and added by aseptic powder addition or by sterile filtration if the drug is water soluble. The remaining components are dissolved separately in approximately ½ of the final weight of water, added to the polymer mixture by sterile filtration (0.22 um filter) and stirred for 10 minutes. The mixture is adjusted to the desired pH with 10N sodium hydroxide while stirring, brought to final weight with water and by sterile filtration and aseptically filled into multi-dose containers. The pH of each formulation is about 6, and the viscosity and osmolality is about 1500 cps and about 280 mOsm/kg, respectively.

The following table shows the compositions of examples of aqueous polymeric suspensions comprising the oxazolidinone compound of formula IV as well as controls lacking the compound of formula IV.

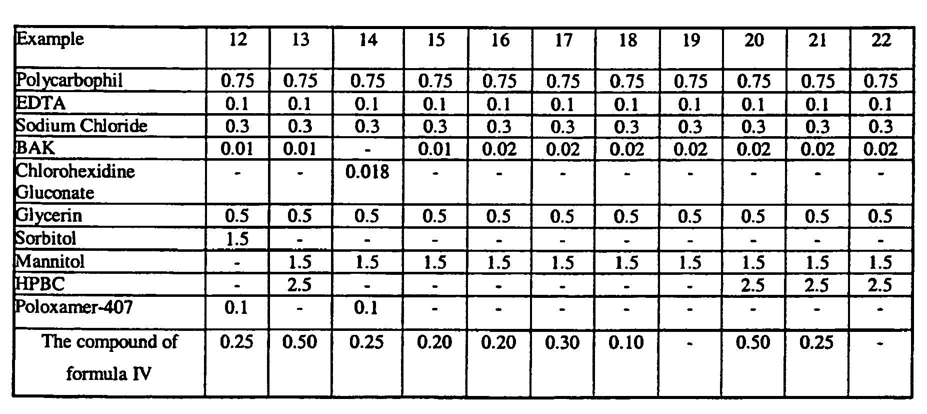

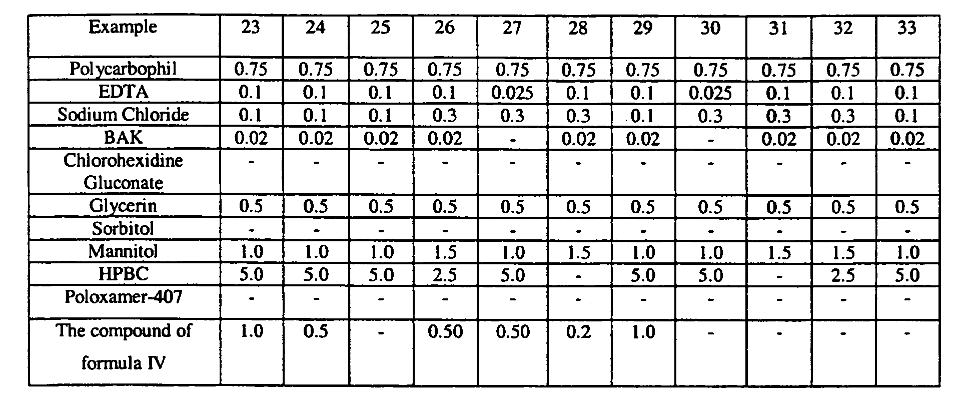

## Claims

1. An aqueous polymeric suspension comprising water, 0,05% to 5,0% of an oxazolidinone antibiotic, 0,1 to 10% of a polymeric suspending agent and a solubility enhancer, wherein said polymeric suspending agent comprises a water-swellable water-insoluble crosslinked carboxy-vinyl polymer.

2. The suspension of claim 1, wherein said suspension comprises 0,1% to 2.0% of said oxazolidinone antibiotic and 0,5% to 6.5% of said polymeric suspending agent.

3. The suspension of claim 1, wherein said polymeric suspending agent further comprises at lesat 90% acrylic acid monomers and 0.1% to 5% crosslinking agent.

4. The suspension of claim 3, wherein said crosslinking agent comprises a difunctional crosslinking agent.

5. The suspension of claim 4, wherein said crosslinking agent is selected from the group consisting of divinyl glycol, 2,3-dihydroxyhexa-1,5-diene, 2,5dimethyl-1,5-hexadiene, divinylbenzene, N,N-diallylacrylamide, N,N-diallylmethacrylamide, and mixtures thereof.

6. The suspension of claim 4, wherein said crosslinking agent is selcted from the group consisting of 2,3-dihydroxyhexa-1,5diene and 2,3-dimethylhexa-1,5-diene.

7. The suspension of claim 1, wherein said polymeric suspending agent further comprises a polycarbophil.

8. The suspension of claim 7, wherein said suspension comprises 0.5% to 1.0% of said polycarbophil.

9. The suspension of claim 1, further comprising an additional medicament.

10. The suspension of claim 9, wherein said additional medicament is selected from the group consisting of antibiotics, antivirals, antifungals, anesthetics, anti-inflammatory agents, and anti-allergic agents.

11. The suspension of claim 10, wherein said additional medicament is about 0.01% to about 5.0% of said suspension.

12. The suspensioon of claim 1, wherein said polymeric suspending agent has a monodisperse particle size distribution.

13. An aqueous polymeric suspension comprising water, 0,05% to 5.0% of an oxazolidinone antibiotic, 0.1 to 10% of a polymeric suspending agent and a solubility enhancer, wherein said polymeric suspending agent comprises a polycarbophil.

14. The suspension of claim 13, wherein said suspension comprises 0.1% to 2.0% of said oxazolidinone antibiotic and 0.5% to 6.5% of said polycarbophil.

15. The suspension of claim 14, wherein said suspension comprises 0.6% to 0.8% of said polycarbophil.

16. The suspension of claim 13, wherein said polymeric suspending agent has a monodisperse particle size distribution.

17. The suspension of claim 13, further comprising an additional medicament.

18. The suspension of claim 17, wherein said additional medicament is selected from the group consisting of antibiotics, antivirals, antifungals, anesthetics, anti-inflammatory agents, and anti-allergic agents.

19. The suspension of claim 18, wherein said additional medicament is about 0.01% to about 5.0% of said suspension.

20. The suspension of claim 13, further comprising 0.1% to 5% difunctional crosslinking agent.

21. The suspension of claim 20, wherein said crosslinking agent is selected from the group consisting of divinyl glycol, 2,3-dihydroxyhexa-1,5-diene, 2,5-dimethyl-1,5-hexadiene, divinylbenzene, N,N-diallylacrylamide, N,N-diallylmethacrylamide, and mixtures thereof.

22. The suspension of claim 20, wherein said crosslinking agent is selected from the group consisting of 2,3-dihydroxyhexa-1,5-diene and 2,3-dimethylhexa-1,5-diene.

23. The suspension of claim 1 or 13, wherein the solubility enhancer is a cyclodextrin.

24. A composition comprising water, a polymeric suspending agent, a solubility enhancer and a compound of formula III: 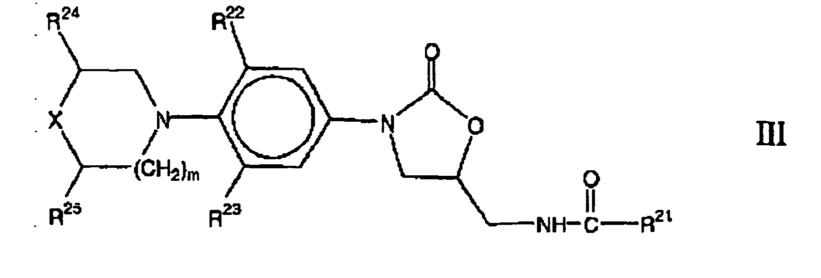
X is O, S, SO, SO₂SNR⁴⁰ or S(O)NR⁴⁰
R²¹ is hydrogen, C₁-C₆ alkyl unsubstituted or substituted with one or more of the following: F, Cl, hydroxy, C₁-C₈ alkoxy, C₁-C₈ acyloxy or -O-CH₂-phenyl; C₁-C₆ cycloalkyl, amino, C₁-C₈ alkyamino, C₁-C₈ dialkylamino or C₁-C₈ alkoxy;
R²⁴ is H; CH₃ CN, CO₂H, CO₂R, or (CH₂)ₘR⁴¹ except when X is not O then R²⁴ is H;
R²² and R²³ are each and independently H, F, or Cl;
R²⁵ is H except when X is O and R²⁴ is CH₃ then R²⁵ can be H or CH₃;
R⁴⁰ is independently H, C₁-C₄ alkyl (unsubstituted or substituted with chloro, fluoro, hydroxy, C₁-C₈ alkoxy, amino, C₁-C₈ alkylamino, or C₁-C₈ dialkyamino) or p-toluenesulfonyl;
R⁴¹ is hydrogen, OH, OR²¹, OCOR²¹, NH₂, NHCOR²¹ or N (R⁴⁰)₂ and m is 0, 1 or 2.

25. The composition of claim 24, wherein the compound of formula III has the following formula:

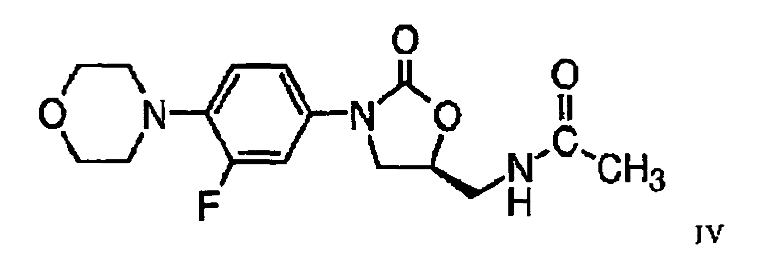

26. The composition of claim 25, wherein said composition comprises 0.1 to 5% of said compound of formula IV.

27. The composition of claim 26, wherein said composition comprises 0.2 to 4% of said compound of formula IV.

28. The composition of claim 24, further comprising a difunctional crosslinking agent.

29. The composition of claim 28, wherein said crosslinking agent is selected from the group consisting of divinyl glycol, 2,3-dihydroxyhexa-1,5-diene, 2,5-dimethyl-1,5-hexadiene, divinylbenzene, N,N-diallylacrylamide, N,N-diallylmethacrylamide, and mixtures thereof.

30. The composition of claim 29, wherein said crosslinking agent is selected from the group consisting of 2,3-dihydroxyhexa-1,5-diene and 2,3-dimethylhexa-1,5-diene.

31. The composition of claim 24, further comprising an additional medicament.

32. The composition of claim 31, wherein said additional medicament is selected from the group consistinig of antibiotics, antivirals, antifungals, anesthetics, anti-inflammatory agents, and anti-allergic agents.

33. The composition of claim 32, wherein said additional medicament comprises about 0.01% to about 5.0% of said composition.

34. The composition of claim 24, wherein said polymeric suspending agent has a monodisperse particle size distribution.

35. The composition of claim 24, wherein said polymeric suspending agent comprises a polycarbophil.

36. The composition of claim 35, wherein said composition comprises 0.5% to 6.5% of said polycarbophil.

37. The composition of claim 36, wherein said composition comprises 0.6% to 0.8% of said polycarbophil.

38. The composition of claim 24, wherein said polymeric suspending agent comprises a water-swellable, water-insoluble crosslinked carboxy-vinyl polymer.

39. The composition of claim 24, wherein said polymeric suspending agent comprises a cellulosic polymer.

40. The composition of claim 24, wherein said polymeric suspending agent comprises at least 90% acrylic acid monomers.

41. A composition comprising water, an oxazolidinone, a polymeric suspending agent, and a solubility enhancer, wherein said suspension comprises 0.1 to 5.0% of a compound of formula III and wherein said solubility enhancer is a cyclodextrin: 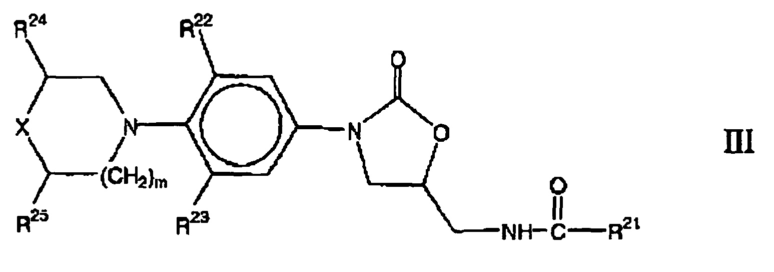 wherein
X is O, S, SO, SO₂SNR⁴⁰ or S(O)NR⁴⁰
R²¹ is hydrogen, C₁-C₆ alkyl unsubstituted or substituted with one or more of the following: F, C1, hydroxy, C₁-C₈ alkoxy, C₁-C₈ acyloxy or -O-CH₂-phenyl; C₁-C₆ cycloalkyl, amino, C₁-C₈ alkyamino, C₁-C₈ dialkylamino or C₁-C₈ alkoxy;
R²⁴ is H; CH₃ CN, CO₂H, CO₂R, or (CH₂)ₘR⁴¹ except when X is not O then R²⁴ is H;
R²² and R²³ are each and independently H, F, or Cl;
R²⁵ is H except when X is O and R²⁴ is CH₃ then R²⁵ can be H or CH₃;
R⁴⁰ is independently H, C₁-C₄ alkyl (unsubstituted or substituted with chloro, fluoro, hydroxy, C₁-C₈ alkoxy, amino, C₁-C₈ alkylamino, or C₁-C₈ dialkyamino) or p-toluenesulfonyl;
R⁴¹ is hydrogen, OH, OR²¹, OCOR²¹, NH₂, NHCOR²¹ or N(R⁴⁰)₂ and m is 0, 1 or 2.

42. The composition of claim 41, wherein the compound of formula III has the following formula:

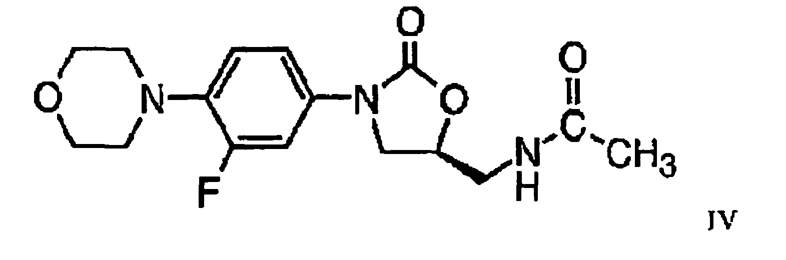

43. The composition of claim 42, wherein said composition comprisses 0.2 to 4.0% of said compound of formula IV and 0.1% to 20% of said cyclodextrin, wherein said cyclodextrin is hydroxypropyl-β-cyclodextrin.

44. The composition of claim 41, further comprising a difunctional crosslinking agent.

45. The composition of claim 44, wherein said crosslinking agent is selected from the group consisting of divinyl glycol, 2,3-dihydroxyhexa-1,5-diene, 2,5-dimethyl-1,5-hexadiene, divinylbenzene, N,N-diallylacrylamide, N,N-diallylmethacrylamide, and mixtures thereof.

46. The composition of claim 45, wherein said crosslinking agent is selected from the group consisting of 2,3-dihydroxyhexa-1,5-diene and 2,3-dimethylhexa-1,5-diene.

47. The composition of claim 41, further comprising an additional medicament.

48. The composition of claim 47, wherein said additional medicament is selected from the group consisting of antibiotics, antivirals, antifungals, anesthetics, anti-inflammatory agents, and anti-allergic agents.

49. The composition of claim 48, wherein said additional medicament is about 0,01% to about 5.0% of said composition.

50. The composition of claim 41, wherein said polymeric suspending agent has a monodisperse particle size distribution.

51. The composition of claim 41, wherein said polymeric suspending agent comprises a cellulosic polymer.

52. The composition of claim 41, wherein said polymeric suspending agent comprises at least 90% acrylic acid monomers.

53. The composition of claim 41, wherein said cyclodextrin is an α-cyclodextrin.

54. The composition of claim 41, wherein said cyclodextrin is a β-cyclodextrin.

55. The composition of claim 41, wherein said cyclodextrin is a Y-cyclodextrin.

56. Use of an aqueous polymeric suspension comprising water, 0,05% to 5,0% of an oxazolidinone antibiotic, 0,1 to 10% of a polymeric suspending agent and a solubility enhancer, wherein said polymeric suspending agent comprises a water-swellable water-insoluble crosslinked carboxy-vinyl polymer, for the preparation of a pharmaceutical composition for treating or preventing infection in a tissue of the eye.

## Patentansprüche

1. Wässrige Polymersuspension umfassend Wasser, 0,05% bis 5,0% eines Oxazolidinon-Antibiotikums, 0,1 bis 10% eines Polymer-Absetzverhütungsmittels und eines Löslichkeitsverstärkers, wobei das Polymer-Absetzverhütungsmittel ein in Wasser quellbares, wasserunlösliches vernetztes Carboxyvinylpolymer umfasst.

2. Suspension gemäß Anspruch 1, wobei die Suspension 0,1% bis 2,0% des Oxazolidinon-Antibiotikums und 0,5% bis 6,5% des Polymer-Absetzverhütungsmittels umfasst.

3. Suspension gemäß Anspruch 1, wobei das Polymer-Absetzverhütungsmittel weiter mindestens 90% Acrylsäuremonomere und 0,1% bis 5% Vernetzungsmittel umfasst.

4. Suspension gemäß Anspruch 3, wobei das Vernetzungsmittel ein zweiwertiges Vernetzungsmittel umfasst.

5. Suspension gemäß Anspruch 4, wobei das Vernetzungsmittel ausgewählt ist aus der Gruppe, die aus Divinylglycol, 2,3-Dihydroxyhexa-1,5-dien, 2,5-Dimethyl-1,5-hexadien, Divinylbenzol, N,N-Diallylacrylamid, N,N-Diallylmethacrylamid und Gemischen daraus besteht.

6. Suspension gemäß Anspruch 4, wobei das Vernetzungsmittel ausgewählt wird aus der Gruppe, die aus 2,3-Dihydroxyhexa-1,5-dien und 2,3-Dimethylhexa-1,5-dien besteht.

7. Suspension gemäß Anspruch 1, wobei das Polymer-Absetzverhütungsmittel weiter ein Polycarbophil umfasst.

8. Suspension gemäß Anspruch 7, wobei die Suspension 0,5% bis 1,0% des Polycarbophils umfasst.

9. Suspension gemäß Anspruch 1, die weiter ein zusätzliches Medikament umfasst.

10. Suspension gemäß Anspruch 9, wobei das zusätzliche Medikament ausgewählt ist aus der Gruppe bestehend aus Antibiotika, Antivirusmitteln, Fungiziden, Anästhetika, anti-entzündlichen Mitteln und anti-allergischen Mitteln.

11. Suspension gemäß Anspruch 10, wobei das zusätzliche Medikament ungefähr 0,01% bis ungefähr 5,0% der Suspension ausmacht.

12. Suspension gemäß Anspruch 1, wobei das Polymer-Absetzverhütungsmittel eine monodisperse PartikelGrößenverteilung hat.

13. Wässrige Polymersuspension umfassend Wasser, 0,05% bis 5,0% eines Oxazolidinon-Antibiotikums, 0,1 bis 10% eines Polymer-Absetzverhütungsmittel und eines Löslichkeitsverstärkers, wobei das Polymer-Absetzverhütungsmittel eine Polycarbophil umfasst.

14. Suspension gemäß Anspruch 13, wobei die Suspension 0,1% bis 2,0% des Oxazolidinon-Antibiotikums und 0,5% bis 6,5% des Polycarbophils umfasst.

15. Suspension gemäß Anspruch 14, wobei die Suspension 0,6% bis 0,8% des Polycarbophils umfasst.

16. Suspension gemäß Anspruch 13, wobei das Polymer-Absetzverhütungsmittel eine monodisperse PartikelGrößenverteilung hat.

17. Suspension gemäß Anspruch 13, weiter umfassend ein zusätzliches Medikament.

18. Suspension gemäß Anspruch 17, wobei das zusätzliche Medikament ausgewählt ist aus der Gruppe bestehend aus Antibiotika, Antivirusmitteln, Fungiziden, Anästhetika, anti-entzündlichen Mitteln und anti-allergischen Mitteln.

19. Suspension gemäß Anspruch 18, wobei das zusätzliche Medikament ungefähr 0,01% bis ungefähr 5,0% der Suspension ausmacht.

20. Suspension gemäß Anspruch 13, weiter umfassend 0,1% bis 5% eines zweiwertigen Vernetzungsmittels.

21. Suspension gemäß Anspruch 20, wobei das Vernetzungsmittel ausgewählt ist aus der Gruppe, die aus Divinylglycol, 2,3-Dihydroxyhexa-1,5-dien, 2,5-Dimethyl-1,5-hexadien, Divinylbenzol, N,N-Diallylacrylamid, N,N-Diallylmethacrylamid und Gemischen daraus besteht.

22. Suspension gemäß Anspruch 20, wobei das Vernetzungsmittel ausgewählt wird aus der Gruppe, die aus 2,3-Dihydroxyhexa-1,5-dien und 2,3-Dimethylhexa-1,5-dien besteht.

23. Suspension gemäß Anspruch 1 oder 13, wobei der Löslichkeitsverstärker ein Cyclodextrin ist.

24. Zusammensetzung umfassend Wasser, ein Polymer-Absetzverhütungsmittel, einen Löslichkeitsverstärker und eine Verbindung der Formel (III): 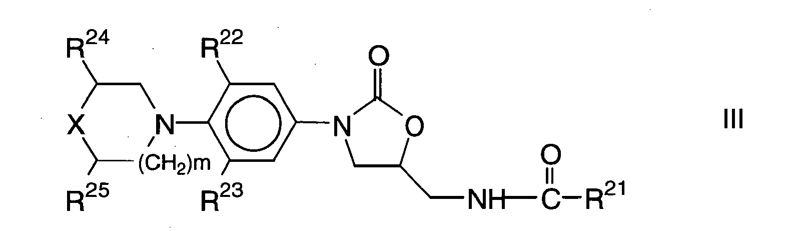 worin
X O, S, SO, SO₂SNR⁴⁰ oder S(O)NR⁴⁰ ist
R²¹ Wasserstoff, C₁-C₆-Alkyl, nicht substituiert oder substituiert mit einem oder mehreren der folgenden: F, Cl, Hydroxy, C₁-C₈-Alkoxy, C₁-C₈-Acyloxy oder -O-CH₂-phenyl; C₁-C₆-Cycloalkyl, Amino, C₁-C₈₋Alkylamino, C₁-C₈-Dialkylamino oder C₁-C₈-Alkoxy ist;
R²⁴ ist H; CH₃, CN, CO₂H CO₂R oder (CH₂)ₘR⁴¹, ausgenommen, wenn X nicht O ist, dann ist R²⁴ H;
R²² und R²³ sind jeweils und unabhängig H, F, oder Cl;
R²⁵ ist H, ausgenommen wenn X O ist und R²⁴ CH₃ ist, dann kann R²⁵ H oder CH₃ sein;
R⁴⁰ ist unabhängig H, C₁-C₄-Alkyl (nicht substituiert oder substituiert mit Chlor, Fluor, Hydroxy, C₁-C₈₋Alkoxy, Amino, C₁-C₈-Alkylamino oder C₁-C₈₋Dialkylamino) oder p-Toluolsulfonyl;
R⁴¹ ist Wasserstoff, OH, OR²¹, OCOR²¹, NH₂, NHCOR²¹ oder N(R⁴⁰)₂ und m ist 0, 1 oder 2.

25. Zusammensetzung gemäß Anspruch 24, wobei die Verbindung der Formel III die folgende Formel hat:

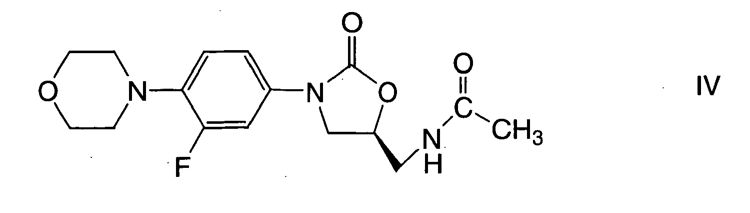

26. Zusammensetzung gemäß Anspruch 25, wobei die Zusammensetzung 0,1 bis 5% der Verbindung der Formel IV umfasst.

27. Zusammensetzung gemäß Anspruch 26, wobei die Zusammensetzung 0,2 bis 4% der Verbindung der Formel IV umfasst.

28. Zusammensetzung gemäß Anspruch 24, weiter umfassend ein zweiwertiges Vernetzungsmittel.

29. Zusammensetzung gemäß Anspruch 28, wobei das Vernetzungsmittel aus der Gruppe ausgewählt ist, die aus Divinylglycol, 2,3-Dihydroxyhexa-1,5-dien, 2,5-Dimethyl-1,5-hexadien, Divinylbenzol, N,N-Diallylacrylamid, N,N-Diallylmethacrylamid und Gemischen daraus besteht.

30. Suspension gemäß Anspruch 29, wobei das Vernetzungsmittel aus der Gruppe ausgewählt ist, die aus 2,3-Dihydroxyhexa-1,5-dien und 2,3-Dimethylhexa-1,5-dien besteht.

31. Zusammensetzung gemäß Anspruch 24, die weiter ein zusätzliches Medikament umfasst.

32. Zusammensetzung gemäß Anspruch 31, wobei das zusätzliche Medikament ausgewählt ist aus der Gruppe bestehend aus Antibiotika, Antivirusmitteln, Fungiziden, Anästhetika, anti-entzündlichen Mitteln und anti-allergischen Mitteln.

33. Zusammensetzung gemäß Anspruch 32, wobei das zusätzliche Medikament ungefähr 0,01% bis ungefähr 5,0% der Zusammensetzung umfasst.

34. Zusammensetzung gemäß Anspruch 24, wobei das Polymer-Absetzverhütungsmittel eine monodisperse PartikelGrößenverteilung hat.

35. Zusammensetzung gemäß Anspruch 24, wobei das Polymer-Absetzverhütungsmittel ein Polycarbophil umfasst.

36. Zusammensetzung gemäß Anspruch 35, wobei die Zusammensetzung 0,5% bis 6,5% des Polycarbophils umfasst.

37. Zusammensetzung gemäß Anspruch 36, wobei die Zusammensetzung 0,6% bis 0,8% des Polycarbophils umfasst.

38. Zusammensetzung gemäß Anspruch 24, wobei das Polymer-Absetzverhütungsmittel ein durch Wasser quellbares, wasserunlösliches vernetztes Carboxyvinylpolymer umfasst.

39. Zusammensetzung gemäß Anspruch 24, wobei das Polymer-Absetzverhütungsmittel ein Zellulosepolymer umfasst.

40. Zusammensetzung gemäß Anspruch 24, wobei das Polymer-Absetzverhütungsmittel mindestens 90% Acrylsäuremonomere umfasst.

41. Zusammensetzung umfassend Wasser, ein Oxazolidinon, ein Polymer-Absetzverhütungsmittel, einen Löslichkeitsverstärker, wobei die Suspension 0,1 bis 5.0% einer Verbindung der Formel III umfasst, und wobei der Löslichkeitsverstärker Cyclodextrin ist: 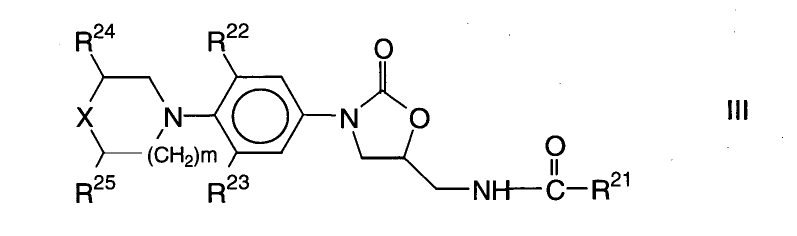 worin
X O, S, SO, SO₂SNR⁴⁰ oder S(O)NR⁴⁰ ist
R²¹ Wasserstoff, C₁-C₆-Alkyl, nicht substituiert oder substituiert mit einem oder mehreren der folgenden: F, Cl, Hydroxy, C₁-C₈-Alkoxy, C₁-C₈-Acyloxy oder -O-CH₂-phenyl; C₁-C₆-Cycloalkyl, Amino, C₁-C₈₋Alkylamino, C₁-C₈-Dialkylamino oder C₁-C₈-Alkoxy ist;
R²⁴ ist H; CH₃, CN, CO₂H, CO₂R, oder (CH₂)ₘR⁴¹, ausgenommen, wenn X nicht O ist, dann ist R²⁴ H;
R²² und R²³ sind jeweils und unabhängig H, F, oder Cl;
R²⁵ ist H, ausgenommen wenn X O ist und R²⁴ CH₃ ist, dann kann R²⁵ H oder CH₃ sein;
R⁴⁰ ist unabhängig H, C₁-C₄-Alkyl (nicht substituiert oder substituiert mit Chlor, Fluor, Hydroxy, C₁-C₈₋Alkoxy, Amino, C₁-C₈-Alkylamino oder C₁-C₈₋Dialkylamino) oder p-Toluolsulfonyl;
R⁴¹ ist Wasserstoff, OH, OR²¹, OCOR²¹, NH₂, NHCOR²¹ oder N(R⁴⁰)₂ und m ist 0, 1 oder 2.

42. Zusammensetzung gemäß Anspruch 41, wobei die Verbindung der Formel III die folgende Formel hat:

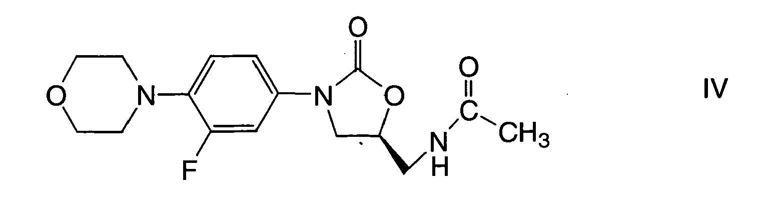

43. Zusammensetzung gemäß Anspruch 42, wobei die Zusammensetzung 0,2 bis 4,0% der Verbindung der Formel IV umfasst und 0,1% bis 20% des Cyclodextrins, wobei das Cyclodextrin Hydroxypropyl-β-cyclodextrin ist.

44. Zusammensetzung gemäß Anspruch 41, weiter umfassend ein zweiwertiges Vernetzungsmittel.

45. Zusammensetzung gemäß Anspruch 44, wobei das Vernetzungsmittel aus der Gruppe ausgewählt ist, die aus Divinylglycol, 2,3-Dihydroxyhexa-1,5-dien, 2,5-Dimethyl-1,5-hexadien, Divinylbenzol, N,N-Diallylacrylamid, N,N-Diallylmethacrylamid und Gemischen daraus besteht.

46. Zusammensetzung gemäß Anspruch 45, wobei das Vernetzungsmittel aus der Gruppe ausgewählt ist, die aus 2,3-Dihydroxyhexa-1,5-dien und 2,3-Dimethylhexa-1,5-dien besteht.

47. Zusammensetzung gemäß Anspruch 41, weiter umfassend ein zusätzliches Medikament.

48. Zusammensetzung gemäß Anspruch 47, wobei das zusätzliche Medikament ausgewählt ist aus der Gruppe bestehend aus Antibiotika, Antivirusmitteln, Fungiziden, Anästhetika, anti-entzündlichen Mitteln und anti-allergischen Mitteln.

49. Zusammensetzung gemäß Anspruch 48, wobei das zusätzliche Medikament ungefähr 0,01% bis ungefähr 5,0% der Zusammensetzung ausmacht.

50. Zusammensetzung gemäß Anspruch 41, wobei das Polymer-Absetzverhütungsmittel eine monodisperse PartikelGrößenverteilung hat.

51. Zusammensetzung gemäß Anspruch 41, wobei das Polymer-Absetzverhütungsmittel ein Zellulosepolymer umfasst.

52. Zusammensetzung gemäß Anspruch 41, wobei das Polymer-Absetzverhütungsmittel mindestens 90% Acrylsäuremonomere umfasst.

53. Zusammensetzung gemäß Anspruch 41, wobei das Cyclodextrin ein α-Cyclodextrin ist.

54. Zusammensetzung gemäß Anspruch 41, wobei das Cyclodextrin ein β-Cyclodextrin ist.

55. Zusammensetzung gemäß Anspruch 41, wobei das Cyclodextrin ein γ-Cyclodextrin ist.

56. Verwendung einer wässrigen Polymersuspension umfassend Wasser, 0,05% bis 5,0% eines Oxazolidinon-Antibiotikums, 0,1 bis 10% eines Polymer-Absetzverhütungsmittels und eines Löslichkeitsverstärkers, wobei das Polymer-Absetzverhütungsmittel ein durch Wasser aufquellbares, wasserunlösliches vernetztes Carboxyvinylpolymer umfasst, zur Herstellung einer pharmazeutischen Zusammensetzung zum Behandeln oder Verhindern einer Infektion in einem Augengewebe.

## Revendications

1. Suspension polymère aqueuse comprenant de l'eau, de 0,05 % à 5,0 % d'un antibiotique de type oxazolidinone, de 0,1 à 10 % d'un agent de suspension polymère et un agent qui augmente la solubilité, dans laquelle ledit agent de suspension polymère comprend un polymère carboxy-vinylique réticulé insoluble dans l'eau et gonflant à l'eau.

2. Suspension selon la revendication 1, dans laquelle ladite suspension comprend de 0,1 % à 2,0 % dudit antibiotique de type oxazolidinone et de 0,5 % à 6,5 % dudit agent de suspension polymère.

3. Suspension selon la revendication 1, dans laquelle ledit agent de suspension polymère comprend en outre au moins 90 % de monomères acide acrylique et de 0,1 % à 5 % d'agent réticulant.

4. Suspension selon la revendication 3, dans laquelle ledit agent réticulant comprend un agent réticulant difonctionnel.

5. Suspension selon la revendication 4, dans laquelle ledit agent réticulant est choisi dans le groupe constitué par le divinylglycol, le 2,3-dihydroxyhexa-1,5-diène, le 2,5-diméthyl-1,5-hexadiène, le divinylbenzène, le N,N-diallylacrylamide, le N,N-diallylméthacrylamide, et leurs mélanges.

6. Suspension selon la revendication 4, dans laquelle ledit agent réticulant est choisi dans le groupe constitué par le 2,3-dihydroxyhexa-1,5-diène et le 2,3-diméthylhexa-1,5-diène.

7. Suspension selon la revendication 1, dans laquelle ledit agent de suspension polymère comprend en outre un polycarbophile.

8. Suspension selon la revendication 7, dans laquelle ladite suspension comprend de 0,5 % à 1,0 % dudit polycarbophile.

9. Suspension selon la revendication 1, comprenant en outre un médicament supplémentaire.

10. Suspension selon la revendication 9, dans laquelle ledit médicament supplémentaire est choisi dans le groupe constitué par des antibiotiques, des antiviraux, des antifongiques, des anesthésiques, des agents anti-inflammatoires et des agents antiallergiques.

11. Suspension selon la revendication 10, dans laquelle ledit médicament supplémentaire constitue environ 0,01 % à environ 5,0 % de ladite suspension.

12. Suspension selon la revendication 1, dans laquelle ledit agent de suspension polymère a une répartition granulométrique monodispersée.

13. Suspension polymère aqueuse comprenant de l'eau, de 0,05 % à 5,0 % d'un antibiotique de type oxazolidinone, de 0,1 à 10 % d'un agent de suspension polymère et un agent qui augmente la solubilité, dans laquelle ledit agent de suspension polymère comprend un polycarbophile.

14. Suspension selon la revendication 13, dans laquelle ladite suspension comprend de 0,1 % à 2,0 % dudit antibiotique de type oxazolidinone et de 0,5 % à 6,5 % dudit polycarbophile.

15. Suspension selon la revendication 14, dans laquelle ladite suspension comprend de 0,6 % à 0,8 % dudit polycarbophile.

16. Suspension selon la revendication 13, dans laquelle ledit agent de suspension polymère a une répartition granulométrique monodispersée.

17. Suspension selon la revendication 13, comprenant en outre un médicament supplémentaire.

18. Suspension selon la revendication 17, dans laquelle ledit médicament supplémentaire est choisi dans le groupe constitué par des antibiotiques, des antiviraux, des antifongiques, des anesthésiques, des agents anti-inflammatoires et des agents antiallergiques.

19. Suspension selon la revendication 18, dans laquelle ledit médicament supplémentaire constitue d'environ 0,01 % à environ 5,0 % de ladite suspension.

20. Suspension selon la revendication 13, comprenant en outre de 0,1 % à 5 % d'un agent réticulant difonctionnel.

21. Suspension selon la revendication 20, dans laquelle ledit agent réticulant est choisi dans le groupe constitué par le divinylglycol, le 2,3-dihydroxyhexa-1,5-diène, le 2,5-diméthyl-1,5-hexadiène, le divinylbenzène, le N,N-diallylacrylamide, le N,N-diallylméthacrylamide, et leurs mélanges.

22. Suspension selon la revendication 20, dans laquelle ledit agent réticulant est choisi dans le groupe constitué par le 2,3-dihydroxyhexa-1,5-diène et le 2,3-diméthylhexa-1,5-diène.

23. Suspension selon la revendication 1 ou 13, dans laquelle l'agent qui augmente la solubilité est une cyclodextrine.

24. Composition contenant de l'eau, un agent de suspension polymère, un agent qui augmente la solubilité et un composé de formule III: 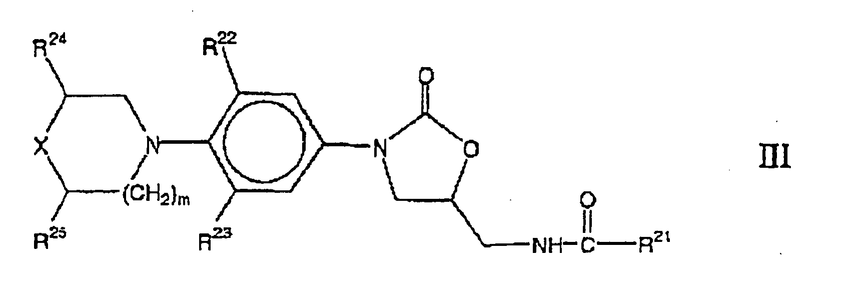 dans laquelle
X est O, S, SO, SO₂SNR⁴⁰ ou S(O)NR⁴⁰;
R²¹ est hydrogène, alkyle en C₁-C₆ non substitué ou substitué par un ou plusieurs des groupes suivants: F, Cl, hydroxy, alcoxy en C₁-C₈, acyloxy en C₁-C₈ ou -O-CH₂-phényle; cycloalkyle en C₁-C₆, amino, alkylamino en C₁-C₈, di(alkyl en C₁-C₈)amino ou alcoxy en C₁-C₈;
R²⁴ est H; CH₃, CN, CO₂H, CO₂R ou (CH₂)ₘR⁴¹, sauf lorsque X n'est pas O, auquel cas R²⁴ est H;
R²² et R²³ sont chacun indépendamment H, F ou Cl;
R²⁵ est H, sauf lorsque X est O et R²⁴ est CH₃, auquel cas R²⁵ peut être H ou CH₃;
R⁴⁰ est indépendamment H, alkyle en C₁-C₄ (non substitué ou substitué par chloro, fluoro, hydroxy, alcoxy en C₁-C₈, amino, alkylamino en C₁-C₈ ou (dialkyl en C₁-C₈)amino) ou p-toluènesulfonyle;
R⁴¹ est hydrogène, OH, OR²¹, OCOR²¹, NH₂, NHCOR²¹ ou N(R⁴⁰)₂ et
m est 0, 1 ou 2.

25. Composition selon la revendication 24, dans laquelle le composé de formule III a la formule suivante:

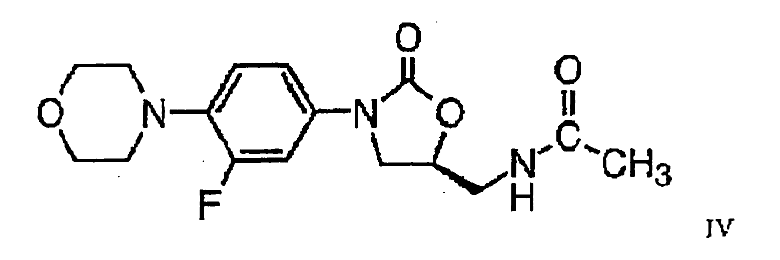

26. Composition selon la revendication 25, dans laquelle ladite composition comprend de 0,1 à 5 % dudit composé de formule IV.

27. Composition selon la revendication 26, dans laquelle ladite composition comprend de 0,2 à 4 % dudit composé de formule IV.

28. Composition selon la revendication 24, comprenant en outre un agent réticulant difonctionnel.

29. Composition selon la revendication 28, dans laquelle ledit agent réticulant est choisi dans le groupe constitué par le divinylglycol, le 2,3-dihydroxyhexa-1,5-diène, le 2,5-diméthyl-1,5-hexadiène, le divinylbenzène, le N,N-diallylacrylamide, le N,N-diallylméthacrylamide, et leurs mélanges.

30. Composition selon la revendication 29, dans laquelle ledit agent réticulant est choisi dans le groupe constitué par le 2,3-dihydroxyhexa-1,5-diène et le 2,3-diméthylhexa-1,5-diène.

31. Composition selon la revendication 24, comprenant en outre un médicament supplémentaire.

32. Composition selon la revendication 31, dans laquelle ledit médicament supplémentaire est choisi dans le groupe constitué par des antibiotiques, des antiviraux, des antifongiques, des anesthésiques, des agents anti-inflammatoires et des agents antiallergiques.

33. Composition selon la revendication 32, dans laquelle ledit médicament supplémentaire constitue d'environ 0,01 % à environ 5,0 % de ladite composition.

34. Composition selon la revendication 24, dans laquelle ledit agent de suspension polymère a une répartition granulométrique monodispersée.

35. Composition selon la revendication 24, dans laquelle ledit agent de suspension polymère comprend un polycarbophile.

36. Composition selon la revendication 35, dans laquelle ladite composition comprend de 0,5 % à 6,5 % dudit polycarbophile.

37. Composition selon la revendication 36, dans laquelle ladite composition comprend de 0,6 % à 0,8 % dudit polycarbophile.

38. Composition selon la revendication 24, dans laquelle ledit agent de suspension polymère comprend un polymère carboxy-vinylique réticulé insoluble dans l'eau et gonflant à l'eau.

39. Composition selon la revendication 24, dans laquelle ledit agent de suspension polymère comprend un polymère cellulosique.

40. Composition selon la revendication 24, dans laquelle ledit agent de suspension polymère comprend au moins 90 % de monomères acide acrylique.

41. Composition comprenant de l'eau, une oxazolidinone, un agent de suspension polymère, et un agent qui augmente la solubilité, dans laquelle ladite suspension comprend de 0,1 à 5,0 % d'un composé de formule III et dans laquelle ledit agent qui augmente la solubilité est une cyclodextrine: 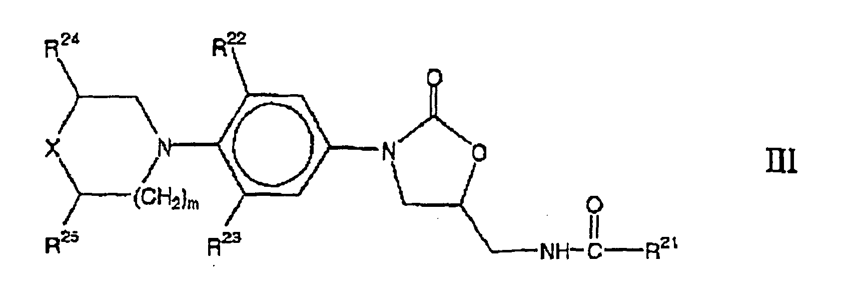 où
X est O, S, SO, SO₂SNR⁴⁰ ou S(O)NR⁴⁰;
R²¹ est hydrogène, alkyle en C₁-C₆ non substitué ou substitué par un ou plusieurs des groupes suivants: F, Cl, hydroxy, alcoxy en C₁-C₈, acyloxy en C₁-C₈ ou -O-CH₂-phényle; cycloalkyle en C₁-C₆, amino, alkylamino en C₁-C₈, di(alkyl en C₁-C₈)amino ou alcoxy en C₁-C₈;
R²⁴ est H; CH₃, CN, CO₂H, CO₂R ou (CH₂)ₘR⁴¹, sauf lorsque X n'est pas O, auquel cas R²⁴ est H;
R²² et R²³ sont chacun indépendamment H, F ou Cl;
R²⁵ est H, sauf lorsque X est O et R²⁴ est CH₃, auquel cas R²⁵ peut être H ou CH₃;
R⁴⁰ est indépendamment H, alkyle en C₁-C₄ (non substitué ou substitué par chloro, fluoro, hydroxy, alcoxy en C₁-C₈, amino, alkylamino en C₁-C₈ ou (dialkyl en C₁-C₈)amino) ou p-toluènesulfonyle;
R⁴¹ est hydrogène, OH, OR²¹, OCOR²¹, NH₂, NHCOR²¹ ou N(R⁴⁰)₂ et
m est 0, 1 ou 2.

42. Composition selon la revendication 41, dans laquelle le composé de formule III a la formule suivante:

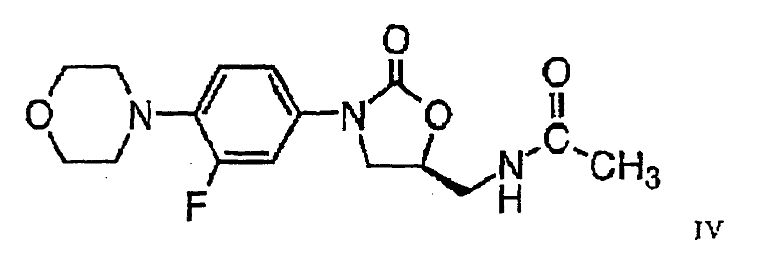

43. Composition selon la revendication 42, dans laquelle ladite composition comprend de 0,2 à 4,0 % dudit composé de formule IV et de 0,1 à 20 % de ladite cyclodextrine, ladite cyclodextrine étant l'hydroxypropyl-β-cyclodextrine.

44. Composition selon la revendication 41, comprenant en outre un agent réticulant difonctionnel.

45. Composition selon la revendication 44, dans laquelle ledit agent réticulant est choisi dans le groupe constitué par le divinylglycol, le 2,3-dihydroxyhexa-1,5-diène, le 2,5-diméthyl-1,5-hexadiène, le divinylbenzène, le N,N-diallylacrylamide, le N,N-diallylméthacrylamide, et leurs mélanges.

46. Composition selon la revendication 45, dans laquelle ledit agent réticulant est choisi dans le groupe constitué par le 2,3-dihydroxyhexa-1,5-diène et le 2,3-diméthylhexa-1,5-diène.

47. Composition selon la revendication 41, comprenant en outre un médicament supplémentaire.

48. Composition selon la revendication 47, dans laquelle ledit médicament supplémentaire est choisi dans le groupe constitué par des antibiotiques, des antiviraux, des antifongiques, des anesthésiques, des agents anti-inflammatoires et des agents antiallergiques.

49. Composition selon la revendication 48, dans laquelle ledit médicament supplémentaire constitue d'environ 0,01 % à environ 5,0 % de ladite composition.

50. Composition selon la revendication 41, dans laquelle ledit agent de suspension polymère a une répartition granulométrique monodispersée.

51. Composition selon la revendication 41, dans laquelle ledit agent de suspension polymère comprend un polymère cellulosique.

52. Composition selon la revendication 41, dans laquelle ledit agent de suspension polymère comprend au moins 90 % de monomères acide acrylique.

53. Composition selon la revendication 41, dans laquelle ladite cyclodextrine est une α-cyclodextrine.

54. Composition selon la revendication 41, dans laquelle ladite cyclodextrine est une β-cyclodextrine.

55. Composition selon la revendication 41, dans laquelle ladite cyclodextrine est une γ-cyclodextrine.

56. Utilisation d'une suspension polymère aqueuse comprenant de l'eau, de 0,05 % à 5,0 % d'un antibiotique de type oxazolidinone, de 0,1 à 10 % d'un agent de suspension polymère et un agent qui augmente la solubilité, dans laquelle ledit agent de suspension polymère comprend un polymère carboxy-vinylique réticulé insoluble dans l'eau et gonflant à l'eau, pour la préparation d'une composition pharmaceutique destinée au traitement ou à la prévention d'une infection dans un tissu de l' oeil.
